(19) Europäisches Patentamt
European Patent Office
Office européen des brevets

(11) Veröffentlichungsnummer: **0 258 686 B1**

(12) # EUROPÄISCHE PATENTSCHRIFT

(45) Veröffentlichungstag der Patentschrift: **10.10.90**

(51) Int. Cl.[5]: **C09B 62/503**, D06P 1/384, C07C 317/14

(21) Anmeldenummer: **87111513.5**

(22) Anmeldetag: **08.08.87**

(54) **Wasserlösliche Triphendioxazin-Verbindungen, Verfahren zu ihrer Herstellung und ihre Verwendung als Farbstoffe.**

(30) Priorität: **13.08.86 DE 3627458**

(43) Veröffentlichungstag der Anmeldung: **09.03.88 Patentblatt 88/10**

(45) Bekanntmachung des Hinweises auf die Patenterteilung: **10.10.90 Patentblatt 90/41**

(84) Benannte Vertragsstaaten: **BE CH DE ES FR GB IT LI**

(56) Entgegenhaltungen:
**EP-A- 0 144 580**
**DE-A- 3 439 755**

(73) Patentinhaber: **HOECHST AKTIENGESELLSCHAFT, Postfach 80 03 20, D-6230 Frankfurt am Main 80(DE)**

(72) Erfinder: **Springer, Hartmut, Dr., Am Erdbeerstein 27, D-6240 Königstein/Taunus(DE)**
Erfinder: **Helmling, Walter, Dr., Fichtestrasse 29, D-6238 Hofheim am Taunus(DE)**
Erfinder: **Schwaiger, Günther, Dr., Johannesallee 41, D-6230 Frankfurt am Main 80(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung liegt auf dem Gebiet der faserreaktiven Farbstoffe.

Aus der europäischen Patentanmeldungs-Veröffentlichung Nr. 0 141 996 A sind faserreaktive Triphendioxazin-Farbstoffe bekannt, die jedoch noch verbesserungswürdige Eigenschaften besitzen.

Es wurden nunmehr neue wasserlösliche Triphendioxazin-Verbindungen entsprechend der allgemeinen Formel (1)

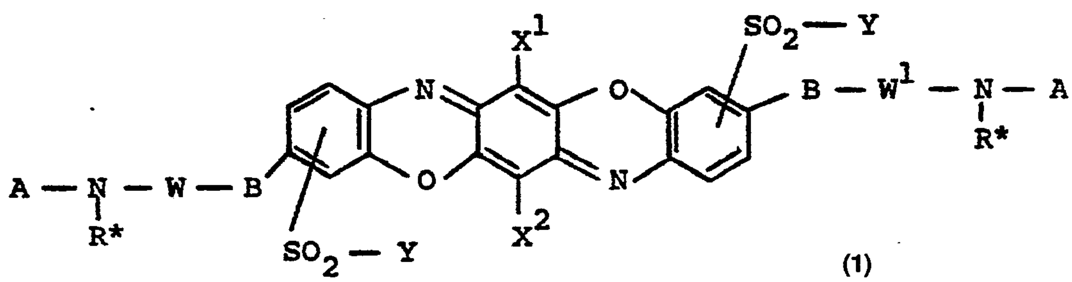

(1)

gefunden, die wertvolle faserreaktive Farbstoffeigenschaften besitzen.

In dieser Formel (1) bedeuten:

B ist ein Sauerstoff- oder Schwefelatom oder eine Aminogruppe der Formel -NH- oder -N(R′)-, in welcher

R′ eine Alkylgruppe von 1 bis 6 C-Atomen, bevorzugt von 1 bis 4 C-Atomen, wie die Methyl- oder Ethylgruppe, ist;

R* ist ein Wasserstoffatom oder eine gegebenenfalls substituierte Alkylgruppe von 1 bis 4 C-Atomen oder ein gegebenenfalls substituierter Arylrest, wie beispielsweise Methyl, Ethyl, Benzyl, Phenethyl, β-Sulfatoethyl, β-Sulfoethyl, Phenyl, Sulfophenyl oder Sulfobenzyl;

W ist ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest, wobei die aliphatischen Reste durch Heterogruppen, bevorzugt 1 oder 2 Heterogruppen, unterbrochen sein können, die aus den Gruppen -O- , -S- , $-SO_2-$ , -CO- , 1,4-Piperidino, -NH- und $-N(R^0)-$ , worin $R^0$ eine der Bedeutungen von R′ hat oder eine Alkanoylgruppe von 2 bis 5 C-Atomen, wie Acetylgruppe, ist, ausgewählt sind, und

$W^1$ hat eine für W angegebene Bedeutung und ist mit W gleich oder von W verschieden, oder

die Gruppierung $-B-W^1-N(R^*)-$ und die Gruppierung -N(R*)-W-B- , zueinander gleich oder voneinander verschieden, stellt jede gemeinsam den bivalenten Rest eines fünf- oder sechsgliedrigen, zwei Stickstoffatome enthaltenden gesättigten Heterocyclus dar oder

die Gruppierung $-B-W^1-$ und die Gruppierung -W-B- , zueinander gleich oder voneinander verschieden, stellt jeder gemeinsam den bivalenten Rest eines fünf- oder sechsgliedrigen, zwei Stickstoffatome enthaltenden gesättigten Heterocyclus dar, der mit einem der beiden Stickstoffatome über eine Alkylengruppe von 2 bis 4 C-Atomen mit der Gruppierung -B(R*)-A bzw. A-N(R*)- verbunden ist;

A ist ein Rest der allgemeinen Formel (2a) oder (2b)

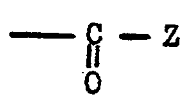

(2a)

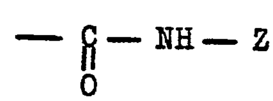

(2b)

in welchen

Z ein Phenylrest oder ein Naphthylrest ist, die beide substituiert sind, wie beispielsweise durch Alkyl von 1 bis 4 C-Atomen, Alkoxy von 1 bis 4 C-Atomen, Chlor, Brom, Nitro, Carbalkoxy von 2 bis 5 C-Atomen, Sulfo und Carboxy, wobei mindestens einer dieser Substituenten eine wasserlöslich machende Gruppe, wie eine Sulfo- oder Carboxygruppe, ist;

$X^1$ ist ein Wasserstoffatom oder ein Halogenatom, wie Chlor- oder Bromatom, eine Alkylgruppe von bis 1 bis 4 C-Atomen, eine Alkoxygruppe von 1 bis 4 C-Atomen oder ein gegebenenfalls substituierter Arylrest;

$X^2$ ist mit $X^1$ gleich oder von $X^1$ verschieden und hat eine der für $X^1$ angegebenen Bedeutungen;

Y ist die Vinylgruppe oder eine Ethylgruppe, die in β-Stellung einen durch ein Alkali eliminierbaren Substituenten enthält;

die Gruppe $-SO_2-Y$ steht bevorzugt in ortho-Stellung zur Gruppe -B-W-N(R*)-A bzw. A-N(R*)-W-B-gebunden;

von den Sulfogruppen, die im Molekül (1) enthalten sein können, enthält das Molekül (1) mindestens zwei.

Die einzelnen, auch zweifach erscheinenden Formelglieder, können zueinander gleiche oder voneinander verschiedene Bedeutungen besitzen; vorzugsweise besitzen sie zueinander gleiche Bedeutungen, weswegen die Verbindungen (1) bevorzugt symmetrisch aufgebaute Verbindungen sind.

Aliphatische Reste sind bevorzugt geradkettige oder verzweigte Alkylengruppen von 1 bis 6 C-Atomen. Substituierte Alkylgruppen sind beispielsweise solche, die durch 1 oder 2 Substituenten aus der Gruppe Chlor, Alkoxy, von 1 bis 4 C-Atomen, Benzoylamino, Sulfobenzoylamino, Alkanoylamino von 2 bis 5 C-Atomen, Hydroxy, Sulfato, Phosphato, Alkanoyloxy von 2 bis 5 C-Atomen, Sulfo, Carboxy oder gegebenenfalls substituiertes Aryl substituiert sein können. Bevorzugte Substituenten sind hiervon die Carboxy- und Sulfogruppen sowie Sulfatogruppen.

Arylreste in den oben genannten oder nachstehend genannten Gruppen sind insbesondere die Phenyl- und Naphthylreste; sie können substituiert sein, wie beispielsweise durch Substituenten aus der Guppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Nitro, Sulfamoyl und Carbamoyl (wobei Sulfamoyl und Carbamoyl durch gegebenenfalls substituiertes Alkyl von 1 bis 4 C-Atomen und/oder gegebenenfalls substituiertes Aryl mono- oder disubstituiert sein können.

Bevorzugt ist W bzw. $W^1$ ein Alkylenrest von 2 bis 6 C-Atomen, insbesondere von 2 bis 4 C-Atomen, oder, falls er durch eine oder zwei Heterogruppen unterbrochen ist, ein Alkylenrest von 2 bis 8 C-Atomen, bevorzugt von 2 bis 6, insbesondere von 2 bis 4 C-Atomen, wobei im Alkylenrest die Heterogruppe bevorzugt ein Sauerstoffatom oder eine Gruppe -NH- ist. Weiterhin sind W bzw. $W^1$ beispielsweise Cycloalkylengruppen von 5 oder 6 C-Atomen mit gegebenenfalls 1 bis 3 Methylgruppen als Substituenten oder zwei solche mit einem Alkylenrest von 1 bis 4 C-Atomen verbundene Cycloalkylengruppen oder ein Alkylenrest von 2 bis 6 C-Atomen, der durch eine solche Cycloalkylengruppe unterbrochen ist.

Reste $W^1$ sind beispielsweise der 1,2-Ethylen-, 1,3-Propylen-, 1,4-Butylen-, 1,5-Pentylen-, 1,6-Hexylen-, 2-Methyl-1,3-propylen-, 1,2-Dimethyl-1,2-ethylen-, 1,3-Dimethyl-1,3-propylen-, 2,2-Dimethyl-1,3-propylen- oder 1,2-, 1,4- und 1,3-Cyclohexylen-Rest oder ein bivalenter Rest der nachstehend angegebenen Formeln (a) bis (s), hiervon bevorzugt der 1,2-Ethylen-, 1,3-Propylen- und 1,4-Butylen-Rest und der Rest der Formel (a):

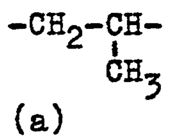
(a)

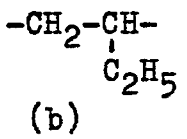
(b)

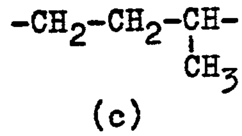
(c)

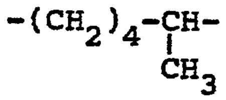 (d)

$-CH_2-CH_2-O-CH_2-CH_2-$ (e)

$-CH_2-CH_2-S-CH_2-CH_2-$ (f)

$-CH_2-CH_2-SO_2-CH_2-CH_2-$ (g)

$-(CH_2)_3-O-CH_2-CH_2-O-(CH_2)_3-$ (h)

$-CH_2-CH_2-NH-CH_2-CH_2-$ (j)

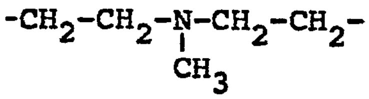 (k)

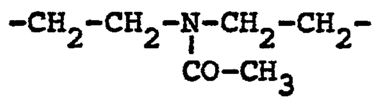 (m)

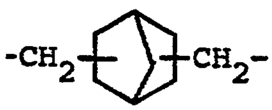 (n)

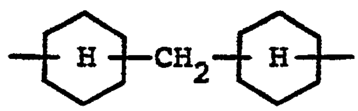 (p)

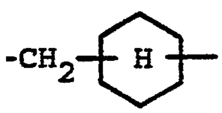 (r)

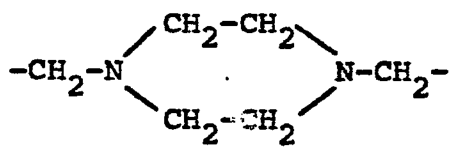 (s).

Die Reste W sind beispielweise die eben für $W^1$ genannten, jedoch "spiegelbildlich" angeordneten Gruppen.

Heterocyclische Gruppierungen, die sich gemeinsam aus den Formelresten -B-$W^1$-N(R*)- bzw. -N(R*)-W-B- bilden, sind beispielsweise der bivalente 1,4-Piperidino-Rest. Gruppierungen mit heterocyclischen Resten, die sich gemeinsam aus dem Formelrest -B-$W^1$- bilden, sind beispielsweise der Rest der Formel (t)

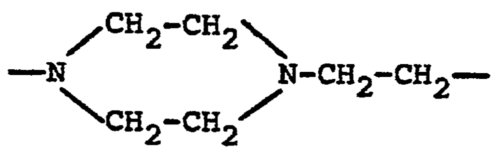

(t)

Gruppierungen mit heterocyclischen Resten, die sich gemeinsam aus dem Formelrest -W-B- bilden, sind beispielsweise ein Rest der Formel (u)

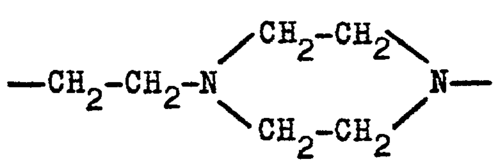

(u).

- Bevorzugt bedeutet weiterhin B die Gruppe NH und R* ein Wasserstoffatom.

Bevorzugt ist in den allgemeinen Formeln (2a) und (2b) der Formelrest Z eine Gruppe der Formel (3a) oder (3b)

(3a) 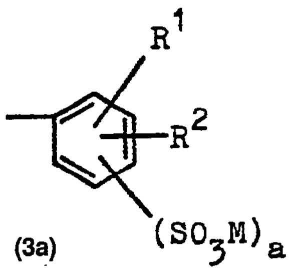 (3b) 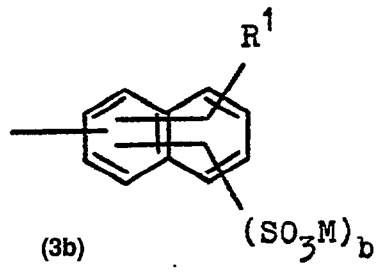

in welchen
M ein Wasserstoffatom oder ein Alkalimetall, wie Natrium, Kalium und Lithium, oder das Äquivalent eines Erdalkalimetalls, wie beispielsweise des Calciums, insbesondere jedoch ein Alkalimetall ist,
$R^1$ ein Wasserstoffatom, eine Alkylgruppe von 1 bis 4 C-Atomen, wie die Ethylgruppe und insbesondere die Methylgruppe, eine Alkoxygruppe von 1 bis 4 C-Atomen, wie die Ethoxygruppe und insbesondere die Methoxygruppe, ein Chlor - oder Bromatom, eine Nitrogruppe, eine Carbalkoxygruppe von 2 bis 5 C-Atomen, wie die Carbomethoxy- oder Carbethoxygruppe, oder eine Sulfogruppe ist und
$R^2$ ein Wasserstoffatom, eine Alkylgruppe von 1 bis 4 C-Atomen, wie die Ethylgruppe und insbesondere die Methylgruppe, eine Alkoxygruppe von 1 bis 4 C-Atomen, wie die Ethoxygruppe und insbesondere die Methoxygruppe, ein Chloratom, eine Carboxygruppe oder eine Sulfogruppe bedeutet,
a für die Zahl Null oder 1 steht, wobei a zwingend 1 ist, falls $R^1$ oder $R^2$ keine der angegebenen wasserlöslich machenden Gruppen bedeutet, und
b die Zahl Null, 1 oder 2 bedeutet, wobei b zwingend die Zahl 1 oder 2 ist, falls $R^1$ keine der genannten wasserlöslichmachenden Gruppen darstellt.

Sofern a bzw. b für die Zahl Null steht, bedeutet diese Gruppe ein Wasserstoffatom.

Bevorzugt sind die Formelreste $X^1$ und $X^2$, zueinander gleich oder voneinander verschieden, bevorzugt zueinander gleich, jedes ein Wasserstoffatom oder insbesondere ein Halogenatom, wie ein Bromatom und insbesondere ein Chloratom.

Bevorzugt ist $R^1$ ein Wasserstoffatom, die Methyl-, Ethyl-, Methoxy-, Ethoxy- oder Sulfogruppe oder ein Chlor- oder Bromatom. Bevorzugt ist $R^2$ ein Wasserstoffatom, die Methoxy-, Ethoxy-, Carboxy- oder Sulfogruppe oder ein Chloratom, und bevorzugt ist a die Zahl 1 und b die Zahl 1 oder 2.

Von den Gruppen Z sind insbesondere hervorzuheben: ein durch 1, 2 oder 3 Sulfogruppen substituierter Naphth-2-yl-Rest, ein durch 1 Bromatom und 1 oder 2 Sulfogruppen substituierter Phenylrest, ein durch 1 oder 2 Ethoxygruppen und 1 oder 2 Sulfogruppen substituierter Phenylrest, ein durch 1 Chloratom und 1, 2 oder 3 Sulfogruppen substituierter Naphth-2-yl-Rest ein durch 1 oder 2 Carb oxygruppen substituierter Phenylrest, insbesondere jedoch ein durch 1 oder 2 Sulfogruppen substituierter Phenylrest, ein durch 1, 2 oder 3 Sulfogruppen substituierter Naphth-1-yl-Rest, ein durch 1 oder 2 Methylgruppen und 1 oder 2 Sulfogruppen substituierter Phenylrest, ein durch 1 oder 2 Chloratome und 1 oder 2 Sulfogruppen substituierter Phenylrest und ein durch 1 oder 2 Methoxygruppen und 1 oder 2 Sulfogruppen substituierter Phenylrest, wobei in den substituierten Phenylresten die Anzahl der Substituenten nicht mehr als drei ist.

Im vorstehenden und nachfolgenden sind Sulfogruppen Gruppen entsprechend der allgemeinen Formel $-SO_3M$ , Carboxygruppen Gruppen entsprechend der allgemeinen Formel $-COOM$ , Sulfatogruppen Gruppen entsprechend der allgemeinen Formel $-OSO_3M$ , thiosulfatogruppen Gruppen entsprechend der allgemeinen Formel $-S-SO_3M$ und Phosphatogruppen Gruppen entsprechend der allgemeinen Formel $-OPO_3M_2$ , wobei M jeweils die oben angegebene Bedeutung besitzt.

Die neuen Dioxazinverbindungen können sowohl in saurer Form als auch in Form ihrer Salze vorliegen. Bevorzugt sind sie in Form der Alkalisalze und finden auch bevorzugt in Form dieser Salze Verwendung zum Färben (im allgemeinen Sinne und einschließlich des Bedruckens verstanden) von hydroxy- und/oder carbonamidgruppenhaltigen Materialien, insbesondere Fasermaterialien.

Bevorzugte erfindungsgemäße Triphendioxazin-Verbindungen sind solche der allgemeinen Formel (1a)

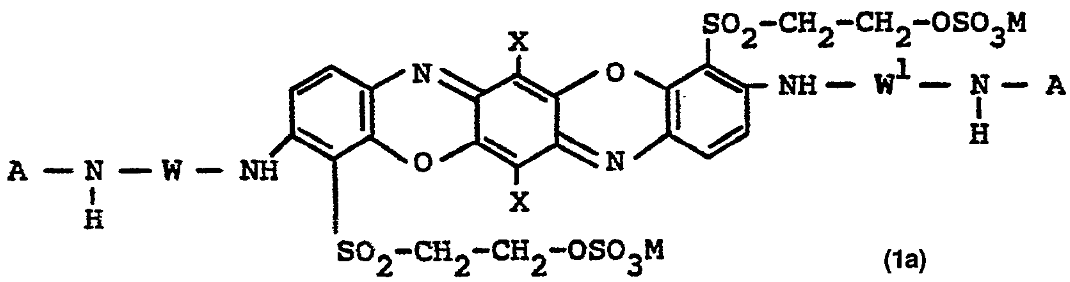

(1a)

in welcher
W und $W^1$ beide eine Alkylengruppe von 2 bis 6 C-Atomen, bevorzugt von 2 bis 4 C-Atomen, bedeuten,
X jedes für ein Bromatom oder insbesondere bevorzugt ein Chloratom steht,
M die oben genannte Bedeutung besitzt und bevorzugt ein Alkalimetall ist und
A beide eine Gruppe der obengenannten Formel (2a) oder (2b) bedeuten, in welchen die Gruppe Z, jeweils zueinander gleich oder voneinander verschieden, eine Gruppe der allgemeinen Formel (3c)

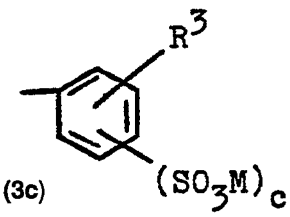

(3c)

bedeutet, in welcher
$R^3$ die Methyl-, Ethyl-, Methoxy- oder Ethoxygruppe oder eine Sulfogruppe oder ein Chlor- oder Bromatom bedeutet, und
c für die Zahl Null, 1 oder 2 steht, wobei c zwingend die Zahl 1 oder 2 ist, falls $R^3$ keine Sulfogruppe bedeutet.

Bevorzugt sind insbesondere von diesen Verbindungen der allgemeinen Formel (1a) diejenigen, in welchen beide X für ein Chloratom stehen, W und $W^1$ beide den 1,2-Ethylenrest bedeuten und beide A jeweils eine Gruppe der Formel (2a) oder (2b) darstellen, in welchen Z jeweils eine Sulfophenylgruppe ist.

Die vorliegende Erfindung betrifft weiterhin Verfahren zur Herstellung der obengenannten und definierten Verbindungen der allgemeinen Formel (1). Diese sind dadurch gekennzeichnet, daß man eine Verbindung der allgemeinen Formel (4)

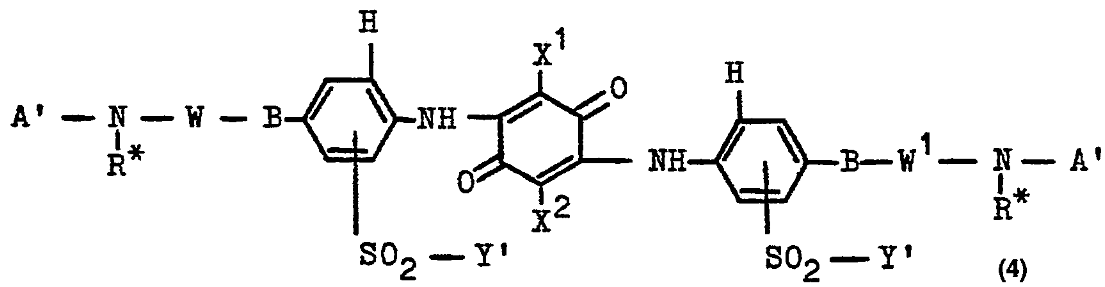

(4)

(in welcher Y' die Vinylgruppe, die β-Hydroxyethyl-Gruppe oder eine Ethylgruppe, die in β-Stellung einen durch ein Alkali eliminierbaren Substituenten, wie einen der für Y genannten, enthält, bevorzugt die β-Hydroxyethyl-Gruppe ist, A' die Bedeutung von A hat, wobei die Gruppen Z jedoch nicht zwingend, auch nicht durch eine wasserlöslich-machende Gruppe, substituiert sein müssen, und B, R*, W, $W^1$, $X^1$ und $X^2$ die obengenannten Bedeutungen haben, und wobei substituierte Alkylgruppen in diesen Resten auch hydroxy-substituierte Alkylgruppen sein können, die Gruppen $-SO_2-Y'$ bevorzugt in ortho-Stellung zur Gruppe B gebunden sind und die Benzolkerne in einer der ortho-Stellungen zur angegebenen Aminogruppe -NH- nicht substituiert sein dürfen) in saurem Medium und vorzugsweise in Gegenwart eines Oxidationsmittels zum Triphendioxazin cyclisiert. Die Umsetzung erfolgt in an und für sich bekannter Verfahrensweise, so beispielsweise in Schwefelsäure oder Schwefeltrioxid enthaltender Schwefelsäure als Reaktionsmedium, wobei als Oxidationsmittel Schwefeltrioxid, Ammonium- oder Alkalipersulfate, Jod oder anorganische Jodverbindungen in Gegenwart von Oleum, Natriumborat, vorzugsweise jedoch Natrium- oder Kaliumperoxidsulfat (entsprechend den Formeln $Na_2S_2O_8$ bzw. $K_2S_2O_8$), verwendet wer-

den. Solche Verfahrensweisen sind bspw. aus der britischen Patentschrift Nr. 1 589 915 und den europäischen Patentanmeldungs-Veröffentlichungen Nrs. 0 141 359A und 0 168 751 A bekannt.

Vorzugsweise führt man die Cyclisierung in konzentrierter Schwefelsäure, wie 96- bis bevorzugt 100%iger Schwefelsäure und insbesondere in Schwefeltrioxid enthaltender Schwefelsäure (Oleum), wie bis zu 50 gew.-%igem Oleum, durch. Die Reaktionstemperatur wird zwischen 0 und 60°C gewählt. Das als Reaktionsmedium und Agenz verwendete Oleum besitzt in der Regel einen Gehalt von 5 bis 40 Gew.-%, bevorzugt 10 bis 20 Gew.-% an Schwefeltrioxid. Bei Zusatzvon Peroxodisulfat als Oxidationsmittel führt man die Cyclisierung zwischen 0 und 40°C, bevorzugt zwischen 15 und 25°C durch. Bei Verwendung von Oleum/Peroxodisulfat soll die Reaktionstemperatur 30°C nicht überschreiten. Bevorzugt ist 10 bis 20%iges Oleum unter Verwendung einer zur Verbindung (4) doppelt molaren Menge Peroxodisulfat. Bei Jod als Oxidationsmittel wird dieses in katalytischen Mengen in 10 bis 50%igem Oleum eingesetzt; hier liegt die Reaktionstemperatur in der Regel zwischen 0 und 40°C.

Gleichzeitig mit der Cyclisierung oder erst nach der Cyclisierungsreaktion können gegebenenfalls vorhandene Hydroxyalkylgruppen, wie beispielsweise die β-Hydroxyethyl-Gruppe des Formelrestes Y', mittels eines Sulfatisierungs- oder Phosphatierungsmittels, wie 96-100%iger Schwefelsäure oder Schwefeltrioxid enthaltender Schwefelsäure bzw. Polyphosphorsäure, in die entsprechenden Sulfatoalkyl- bzw. Phosphatoalkyl-Gruppen verestert werden. Wird also der Ringschluß in Schwefelsäure oder Oleum als Reaktionsmedium ausgeführt, werden Hydroxygruppen, die an einem Alkylrest des Moleküls gebunden sind, wie beispielsweise die oben bereits erwähnten β-Hydroxyethyl-Gruppen des Formelrestes Y' oder Hydroxyalkyl-Gruppen der Formelreste R*, W und $W^1$, in die entsprechenden Sulfatoalkylgruppen übergeführt.

Bei Cyclisierungstemperaturen von oberhalb 15°C, insbesondere oberhalb 25°C, lassen sich mittels 100 %iger Schwefelsäure oder Oleum erfindungsgemäß auch Sulfogruppen in die aromatischen Ringe der Arylreste von R*, $X^1$ , $X^2$ , Z einführen. Die Sulfierungsreaktion kann aber auch nach der Cyclisierung, gegebenenfalls in einem gesonderten Reaktionsschritt beim isolierten Cyclisierungsprodukt (Triphendioxazin), erfolgen. Hier wird die Sulfierungsreaktion in der Regel zwischen 20 und 80°C, bevorzugt zwischen 30 und 70°C, geführt.

Die gleichzeitige oder nachträgliche Sulfierungsreaktion ist eine besonders vorteilhafte Variante des erfindungsgemäßen Verfahrens. Bei dieser erfindungsgemäßen Verfahrensweise erhält man Triphendioxazin-Verbindungen der allgemeinen Formel (1) als Gemisch mit unterschiedlicher Zahl von Sulfogruppen.

Verbindungen der Formel (1) mit Y gleich einer β-Sulfatoethyl-Gruppe können anschließend gemäß bekannten Verfahrensweise in andere erfindungsgemäße Verbindungen der Formel (1) übergeführt werden, in welcher die Y für Vinylgruppen oder Ethylgruppen mit einem anderen in β-Stellung befindlichen alkalisch eliminierbaren Substituenten stehen.

Die Ausgangsverbindungen der allgemeinen Formel (4) können in ebenfalls erfindungsgemäßer Weise durch Umsetzung einer Verbindung der allgemeinen Formel (5)

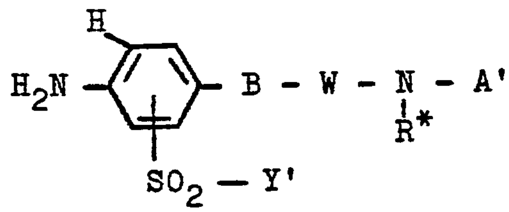

(5)

(worin W auch für $W^1$ steht und Y' die obengenannte Bedeutung besitzt und bevorzugt die β-Hydroxyethyl-Gruppe ist und B, R* und A' die obengenannten Bedeutungen haben, wobei substituierte Alkylgruppen in diesen Resten auch hydroxysubstituierte Alkylgruppen sein können und Arylreste auch von Sulfogruppen frei sein können und die Gruppen $-SO_2-Y'$ bevorzugt in ortho-Stellung zur Gruppe B gebunden sind) mit einer 1,4-Benzochinon-Verbindung der allgemeinen Formel (6)

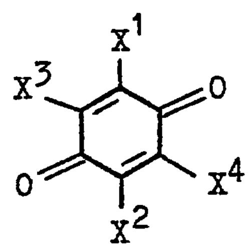

(6)

in welcher $X^1$ und $X^2$ die obengenannten Bedeutungen haben und $X^3$ und $X^4$ zueinander gleich oder voneinander verschieden sind und jedes für ein Wasserstoffatom, eine Alkoxygruppe von 1 bis 4 C-Atomen, wie insbesondere die Methoxygruppe, oder für eine Phenoxygruppe steht oder bevorzugt ein Halogenatom, wie Fluoratom, besonders Bromatom und insbesondere Chloratom, ist, wobei $X^3$ und $X^4$ auch eine zu $X^1$ und $X^2$ gleiche Bedeutung haben können, hergestellt werden.

Die Umsetzung einer Verbindung der allgemeinen Formel (6) oder zweier verschiedener Aminoverbindungen der allgemeinen Formel (5), jeweils in zusammen 2-fach äquivalenter Menge, mit einem Äquivalent einer Verbindung der allgemeinen Formel (6) zur Verbindung der allgemeinen Formel (4) erfolgt analog bekannten Verfahrensweisen, die beispielsweise in den europäischen Patentanmeldungs-Veröffentlichungen Nrs. 0 141 996A und 0 168 751 A erwähnt und beschrieben sind.

Beispielsweise kann die Umsetzung in wäßrigem Medium oder in wäßrig-organischem Medium oder in rein organischem Medium erfolgen, wobei die organischen Lösemittel polare aprotische und protische Lösungsmittel darstellen, wie beispielsweise niedere Alkanole, wie Methanol und Ethanol, und halogenierte Benzole, wie o-Dichlorbenzol. Bevorzugt wird das Chinon (6) jedoch in einem mehr oder weniger stärkeren Überschuß eingesetzt, der in der Regel 5-20 % beträgt, aber auch, abhängig vom gewählten Chinon, bis zu 100 % oder mehr betragen kann. Die Umsetzung der Amine (5) mit den Chinonen (6) kann bei einer Temperatur zwischen 20 und 100°C, vorzugsweise zwischen 50 und 70°C, in Gegenwart eines säurebindenden Mittels, wie beispielsweise eines Alkali- oder Erdalkalicarbonats oder - acetats, so beispielsweise Natriumacetat, Natriumcarbonat oder Natriumbicarbonat, oder eines Alkali- oder Erdalkalihydroxids, wie Natriumhydroxid, oder eines Oxids eines Erdalkalimetalls, wie beispielsweise Magnesiumoxid, durchgeführt werden. Sofern in einem wäßrigen oder wäßrig-organischen Medium gearbeitet wird, wird ein pH-Bereich zwischen 4 und 7, vorzugsweise zwischen 5,5 und 6,5, eingestellt.

Die Anilin-Ausgangsverbindungen der allgemeinen Formel (5) sind bisher hoch nicht bekannt. Die Erfindung betrifft somit auch diese Verbindungen, Verfahren zu deren Herstellung und ihre Verwendung zur Synthese von Farbstoffen. Sie lassen sich analog bekannten Verfahrensweisen der Umsetzung von Aminoverbindungen mit Säurechloriden oder Isocyanaten herstellen, so beispielsweise erfindungsgemäß durch Umsetzung einer Aminoverbindung der allgemeinen Formel (7)

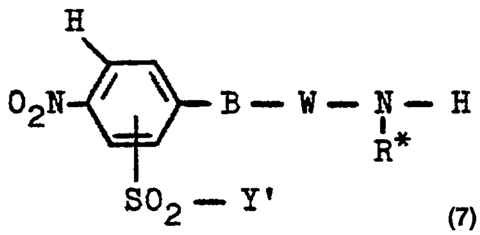

(7)

in welcher Y', B, W und R* die oben genannten Bedeutungen haben, mit einer Verbindung entsprechend einer in den Ansprüchen genannten und definierten Formeln (8A), (8B) und (8C), bevorzugt mit einer Verbindung der allgemeinen Formel (8a), (8b) oder (8c) bzw. (9a) oder (9b)

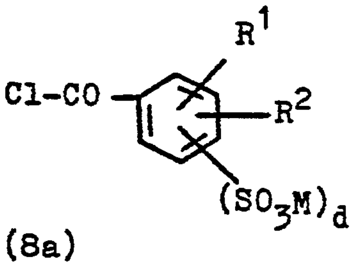

(8a)

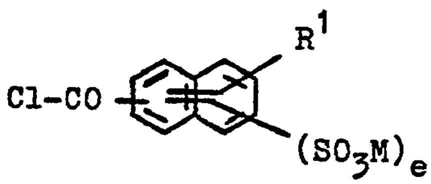

(8b)

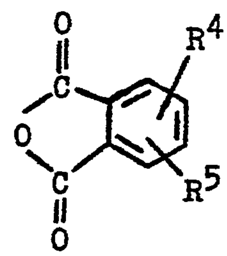

(8c)

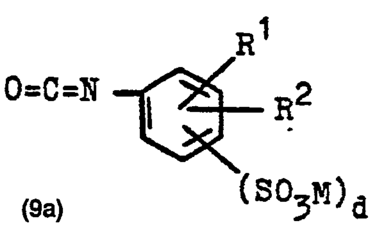

(9a)

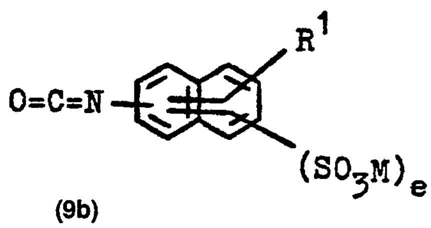

(9b)

in welchen $R^1$ und $R^2$ die oben genannten Bedeutungen haben, d für die Zahl Null, 1 oder 2 steht, e die Zahl Null, 1, 2 oder 3 bedeutet, $R^4$ ein Wasserstoffatom, eine Alkylgruppe von 1 bis 4 C-Atomen, wie Ethylgruppe und insbesondere Methylgruppe, eine Alkoxygruppe von 1 bis 4 C-Atomen, wie Ethoxygruppe und insbesondere Methoxygruppe, ein Chlor- oder Bromatom oder eine Nitrogruppe ist und $R^5$ ein Wasserstoffatom, eine Alkylgruppe von 1 bis 4 C-Atomen, wie die Ethylgruppe und insbesondere Methylgruppe, eine Alkoxygruppe von 1 bis 4 C-Atomen, wie die Ethoxygruppe und insbesondere die Methoxygruppe, oder ein Chloratom ist, umsetzt, und anschließende Reduktion der Nitrogruppe.

Bevorzugt sind von den Verbindungen der Formel (5) solche, in welchen A' einen Rest der Formel (2c) oder (2d)

- CO - Z' (2c)- CO - NH - Z' (2d)

bedeutet, worin Z' ein Rest der Formel (3d), (3e) oder (3f)

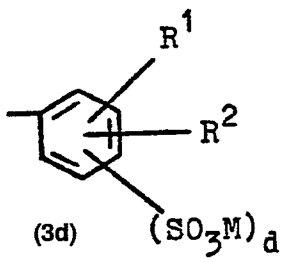
(3d)

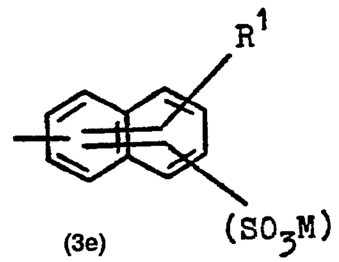
(3e)

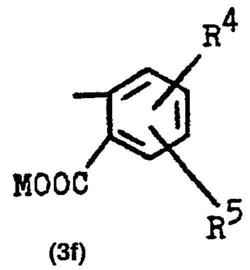
(3f)

ist, in welchen $R^1$, $R^2$, M, d, e, $R^4$ und $R^5$ die obengenannten Bedeutungen haben.

Die Reaktion der Verbindungen der allgemeinen Formel (7) mit den Verbindungen (8) oder (9) erfolgt gemäß den in der Literatur für diese Reaktionsart beschriebenen Verfahrensweisen, wie beispielsweise in wäßrigem Medium oder auch in einem für diese Reaktionsart gängigen organischen Medium, wie in einem Alkanol von 1 bis 6 C-Atomen, wie insbesondere Methanol oder Ethanol, Aceton, Methylethyketon, Dioxan, N,N-Dimethylformamid, N-Methyl-pyrrolidon(2) oder Toluol oder einem Gemisch von Wasser und einem wassermischbaren organischen Lösemittel, bei einer Temperatur zwischen 0 und 60°C und, sofern in wäßrigem oder wäßrig-organischem Medium gearbeitet wird, bei einem pH-Wert zwischen 3 und 12. Bei der Umsetzung der Verbindungen (7) mit den Verbindungen (8) ist es Zweckmäßig, ein übliches säurebindendes Mittel zuzusetzen, um den freiwerdenden Chlorwasserstoff zu binden.

Carbonsäurechloride der allgemeinen Formel (8) sind beispielsweise Benzoylchlorid, 4-Methoxy-benzoylchlorid, 2-Methyl-, 3-Methyl- und 4-Methyl-benzoylchlorid, 4-Chlorbenzoylchlorid, 3-Chlor-benzoylchlorid, 2-Chlor-benzoylchlorid, 4-brom-benzoylchlorid, 3-Brom-benzoylchlorid, 4-Nitro-benzoylchlorid, Naphthalin-1-carbonsäurechlorid, Naphthalin-2-carbonsäurenchlorid, 2,5-Dichlor-benzoylchlorid, 2,4-Dichlor-benzoylchlorid, 3,4-Dichlor-benzoylchlorid, 2-Methoxy-benzoylchlorid, 3-Methoxy-benzoylchlorid, 2-Nitro-4-methyl-benzoylchlorid.

Isocyanate der allgemeinen Formel (9) sind beispielsweise Phenylisocyanat, 4-Chlor-phenylisocyanat, 3-Methyl-phenylisocyanat, 3,5-Dimethyl-phenylisocyanat, 2-Methyl-phenylisocyanat, 3-Chlor-phenylisocyanat, 3,5-Dichlor-phenylisocyanat, 2,3-Dichlor-phenylisocyanat, 3-Chlor-4-methylphenylisocyanat, 1-Naphthyl-isocyanat und 4-Isopropylphenylisocyanat.

Aus den obigen beispielhaften Angaben ist ersichtlich, daß bevorzugt von solchen Verbindungen ausgegangen werden kann, die im aromatischen Rest nicht durch eine Sulfogruppe substituiert sind, da die Einführung einer (oder mehrerer) Sulfogruppen in die aromatischen Reste bevorzugt und verfahrensgemäß vorteilhafter während oder nach der Ringschlußreaktion zum Triphendioxazin-Gerüst mittels Schwefeltrioxid enthaltender Schwefelsäure oder 100%iger Schwefelsäure, wie oben angegeben erfolgt.

Die aus den Reaktionen der Verbindungen (7) mit den Verbindungen (8) bzw. (9) erhaltenen Nitroverbindungen entsprechend einer allgemeinen Formel (10)

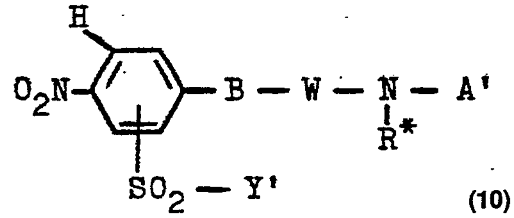
(10)

in welcher Y', B, W, R* und A' die oben genannten Bedeutungen haben, können sodann nach an und für sich üblichen Methoden der Reduktion von aromatischen Nitrogruppen mittels Wasserstoff an einem metallischen Katalysator, wie einem Palladium-, Platin- oder Raney-Nickelkatalysator, unter Druck im Autoklaven oder mittels der Reduktion nach Béchamp bei Verwendung von Eisenspänen zur Verbindung der allgemeinen Formel (5) überführt werden.

Die Ausgangsverbindungen entsprechend der allgemeinen Formel (7) sind bekannt oder können analog hierzu in an und für sich üblicher Weise hergestellt werden, wie beispielsweise nach den Angaben der deutschen Offenlegungsschrift Nr. 3 502 991.

Ausgangsverbindungen der allgemeinen Formel (5) sind beispielsweise: 3-(β-Hydroxyethylsufonyl)-4-N-(β-benzoylamino-ethyl)-amino-anilin, 3-(β-Hydroxyethylsulfonyl)-4-N-(γ-benzoylamino-propyl)-amino-anilin, 3-(β-Hydroxyethylsulfonyl)-4-N-(β-benzoylamino-propyl)-amino-anilin, 3-(β-Hydroxyethylsulfonyl)-4-N-[β-(2'-, 3'- oder 4'-methyl-benzoylamino)-ethylamino]-anilin, 3-(β-Hydroxyethyl-sulfonyl)-4-N-[β-(4'-chlorbenzoylamino)-ethylamino]-anilin, 3-(β-Hydroxyethylsulfo nyl)-4-N-[β-(4'-methoxy-benzoylamino)-ethylamino]-anilin, 3-(β-Hydroxyethylsulfonyl)-4-N-[β(2'-Methoxybenzoylamino)-ethylamino]-anilin, 3-(β-Hydroxyethylsulfonyl)-4-N-{β-[β'-(4'-Methoxy-benzoylamino)-ethoxy]-ethylamino}-anilin, 3-(β-Hydroxyethylsulfonyl)-4-N-[β-(N'-phenylureido)-ethylamino]-anilin, 3-(β-Hydroxyethyl-sulfonyl)-4-N-[β-(N'-phenylureido)-propylamino]-anilin, 3-(β-Hydroxyethylsulfonyl)-4-N-[β-(N'3'-methylphenylureido)-propylamino]-anilin, 3-(β-Hydroxyethylsulfonyl)-4-N-[γ-(N'-2'-methylphenylureido)-propylamino]-anilin, 3-(β-Hydroxyethylsulfonyl)-4-N-{β-[β'-(N'-phenylureido)-ethylsulfonyl]-ethylamino}-anilin und 3-(β-Hydroxyethylsulfonyl)-4-N-[β-(N'-naphth-1'-ylureido)-erthylamino]-anilin.

Die als Ausgangsverbindungen dienenden Benzochinone der allgemeinen Formel (6) sind beispielsweise 2-Methyl-3,5,6-tribrom-1,4-benzochinon, 2-Methoxy-3,5,6-trichlor-1,4-benzochinon, 2,3,5,6-Tetramethoxy-1,4-benzochinon, 2,3,5,6-Tetraphenoxy-1,4-benzochinon, 2-Methyl-3,6-dichlor-1,4-benzochinon, 2,5-Dichlor-1,4-benzochinon und bevorzugt 2,3,5,6-Tetrabrom-1,4-benzochinon und insbesondere 2,3,5,6-Tetrachlor-1,4-benzochinon (Chloranil).

Die Abscheidung und Isolierung der erfindungegemäß hergestellten Verbindungen der allgemeinen Formel (1) aus den syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei dieser Reaktionslösung eine Puffersubstanz zugefügt werden kann.

Die erfindungsgemäßen Verbindungen der allgemeinen Formel (1) - im nachfolgenden als Verbindungen (1) bezeichnet - haben faserreaktive Eigenschaften und besitzen wertvolle Farbstoffeigenschaften. Sie können deshalb zum Färben (einschließlich Bedrucken) von natürlichen, regenerierten oder synthetischen hydroxygruppenhaltigen und/oder carbonamidgruppenhaltigen Materialien, beispielsweise in Form von Flächengebilden, wie Papier und Leder, oder in der Masse, von Polyamid oder Polyurethan, insbesondere aber von solchen Materialien in Faserform, wie Cellulosefasermaterialien, Seide, Wolle und synthetische Polyamid- und Polyurethanfasern, verwendet werden. Auch können die bei der Synthese der Verbindungen (1) anfallenden Lösungen, gegebenenfalls nach Zusatz einer Puffersubstanz, gegebenenfalls auch nach Kozentrierung, direkt als Flüssigpräparation der färberischen Verwendung zugeführt werden.

Die Verbindungen (1) lassen sich, gemäß der erfindungsgemäßen Anwendung, auf den genannten Substraten, insbesondere auf den genannten Fasermaterialien, nach den für wasserlösliche, insbesondere faserreaktive, Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem, gegebenenfalls durch Hitzeeinwirkung und/oder gegebenenfalls durch Einwirkung eines alkalisch wirkenden Mittels, fixiert. Solche Färbe- und Fixierweisen sind in der Literatur zahlreich beschrieben, wie beispielsweise auch in der europäischen Patentanmeldungs-Veröffentlichung Nr. 0 168 751 A.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen der Applikation und Fixierung von faserreaktiven Farbstoffen vorgehen.

Die mit den Verbindungen (1) hergestellten Färbungen und Drucke zeichnen sich durch reine, vorwiegend blaue Farbtöne aus. Insbesondere die Färbungen und Drucke auf Cellulosefasermaterialien besitzen sehr hohe Farbstärken und ebenso sehr gute Lichtechtheiten, einschließlich guter Naßlicht- und Schweißlichtechtheiten, ebenso gute Hypochloritbleich- und Chlorbadewasserechtheiten, weiterhin vorzügliche Naßechtheiten, wie beispielsweise gute Waschechtheiten bei 60 bis 95°C, auch in Gegenwart von Perboraten, saure und alkalische Walk-, Überfärbe- und Schweißechtheiten, Alkali-, Säure-, Wasser- und Seewasserechtheiten, desweiteren eine gute Plissierechtheit, Bügelechtheit und Reibechtheit. Ebenso besitzen sie eine gute Naßliegeechtheit und eine gute Säurelagerbeständigkeit ("acid fading") beim Lagern von feuchten, noch Essigsäure enthaltendem, gefärbtem Material. Desweiteren sind die Färbungen gegen die üblichen Kunstharzappreturen stabil. Ein Teil der Verbindungen (1) sind in der Reinheit des Farbtones und in wichtigen Echtheitseigenschaften mit faserreaktiven Anthrachinonfarbstoffen vergleichbar.

Die nachstehenden Beispiele dienen zur Erläuterung der Erfindung. Die Teile sind Gewichsteile, die Prozentangaben stellen Gewichstsprozente dar, sofern nicht anders vermerkt. Gewichtsteile beziehen sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Natrium- oder Kaliumsalze hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, wie Natrium- oder Kaliumsalze, in die Synthese eingesetzt werden.
Die Absorptionsmaxima ($\lambda_{max}$-Werte) wurden in wäßriger Lösung bestimmt. Die Farbkennzahlen beziehen sich auf Colour Index Hue Indication Chart (Indicator Numbers). Die $^1$H-NMR-Messungen erfolgten in $d_6$-Dimethylsulfoxid mit Tetramethylsilan als innerem Standard, sofern nicht anders angegeben.

Beispiel 1

a) Zu einer Lösung von 60 Teilen 4-(β-Aminoethylamino)-3-(β-hydroxyethylsulfonyl)-nitrobenzol in 150 Teilen N-Methyl-pyrrolidon-(2) gibt man 24 Teile Triethylamin und anschließend stetig 31 Teile Benzoylchlorid, wobei die Reaktionstemperatur auf 45°C ansteigt. Man rührt 3 Stunden nach und rührt den Ansatz sodann auf 1000 Teile Eiswasser. Nach 2 Stunden wird das in reiner Form und hoher Ausbeute angefallene 4-(β-Benzoylaminoethylamino)-3-(β-hydroxyethylsulfonyl)-nitrobenzol abgesaugt und getrocknet; es hat eien Schmelzpunkt von 116-118°C.

b) 50 Teile der unter a) erhaltenen Nitroverbindung werden in 250 Teilen Ethanol gelöst und in Gegenwart eines Nickelkatalysators in einem Autoklaven bei einer Temperatur von bis zu 70°C und einem Druck von 50 bar hydriert. Die Hydrierdauer beträgt etwa vier Stunden. Der Katalysator wird sodann abfiltriert und das Filtrat durch Abdestillation des Lösemittels eingeengt. Die gewünschte Verbindung der Formel

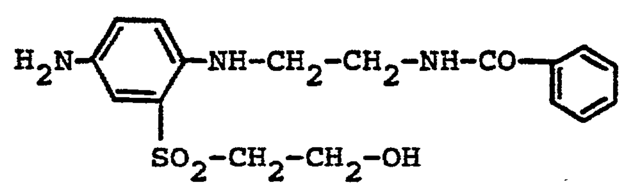

wird in Form eines Öles erhalten.

c) 77 Teile des unter b) synthetisierten 4-(β-Benzoylaminoethylamino)-3-(β-hydroxyethylsulfonyl)-anilins werden in einer Mischung aus 700 Teilen Wasser und 300 Teilen Methanol gelöst; zu dieser auf 60°C erwärmten Lösung werden 27 Teile Chloranil unter Einhaltung eines pH-Wertes von 6 bis 6,5 (bspw. mittels Natriumbicarbonat) eingetragen. Nach 30-minütigem Nachrühren wird das ausgefallene Kondensationsprodukt abgesaugt, zunächst mit wäßrigem Ethanol, sodann mit Wasser gewaschen und unter reduziertem Druck bei 80°C getrocknet.

d) 18 Teile des unter c) synthetisierten Kondensationsproduktes werden in 150 Teile 10 %igem Oleum bei 20°C eingetragen. Der Ansatz wird bis zur vollständigen Lösung gerührt, sodann werden 10 Teile Natriumperoxodisulfat langsam unter Einhaltung einer Reaktionstemperatur von 20 bis 25°C zugegeben. Man rührt noch eine Stunde nach und gießt den Ansazt sodann auf 500 Teile Eis und saugt das ausgefallene Produkt ab. Die erhaltene Triphendioxazin-Verbindung wird in 300 Teilen Wasser angerührt, die Suspension mit Natriumcarbonat auf einen pH-Wert von 6 gestellt, die Triphendioxazin-Verbindung wiederum abgesaugt und getrocknet.

e) 20 Teile der unter d) erhaltenen Triphendioxazin-Verbindung der Formel

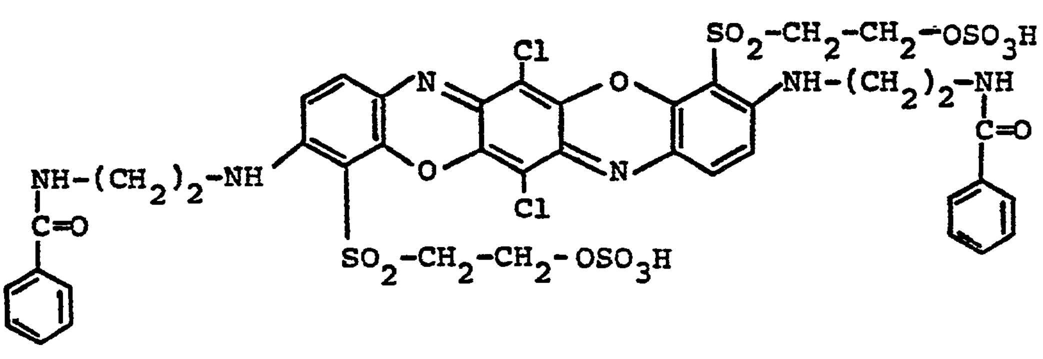

werden in 100 Teilen 20 %igem Oleum eingetragen und während 2 Stunden auf eine Temperatur von 65°C gehalten. Nach dem Erkalten wird der Ansatz auf 1000 Teile Eis gerührt und das ausgefallene Produkt abgesaugt, in 250 Teilen Wasser angerührt und mit Natriumcarbonat auf einen pH-Wert von 6 gestellt; die Lösung wird mit Kaliumchlorid in einer Menge von 25 %, bezogen auf das Volumen der Lösung, versetzt und die ausgefallene erfindungsgemäße Verbindung abgesaugt und bei 60°C unter reduziertem Druck getrocknet.

Man erhält das erfindungsgemäße Alkalimetallsalz (vorwiegend Kaliumsalz) der Verbindung der Formel

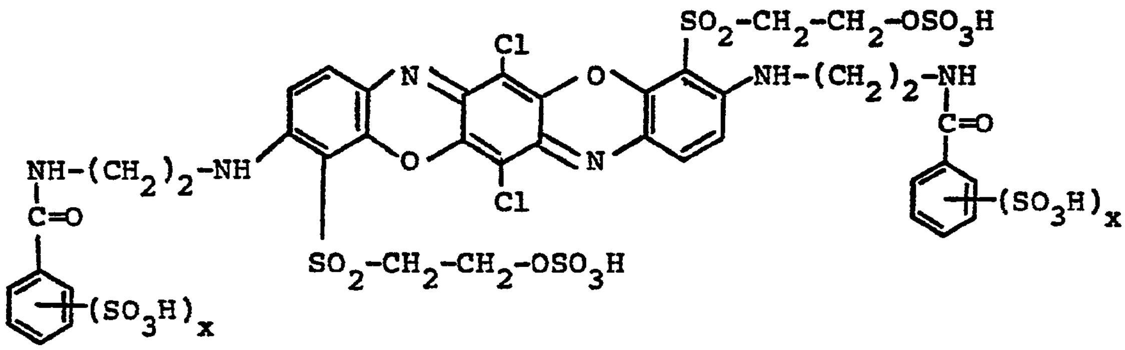

(worin x einen Sulfierungsgrad von ca. 1,2 angeben soll) als elektrolytsalzhaltiges (vorwiegend Kaliumchloridhaltiges) Pulver (die jeweilige β-Sulfatoethylsulfonyl-Gruppe kann auch in der anderen ortho-Stellung zur Aminogruppe gebunden sein, befindet sich jedoch mit größerer Wahrscheinlichkeit in der in der obigen Formel angegebenen Stellung).

Diese erfindungsgemäße Verbindung besitzt sehr gute faserreaktive Farbstoffeigenschaften ($\lambda_{max}$ = 610 nm). Sie färbt die in der Beschreibung genannten Materialien, insbesondere Celulosefasermaterialien, wie Baumwolle, nach den in der Technik üblichen und bekannten Verfahrensweisen der Applikation und Fixierung von faserreaktiven Farbstoffen in farbstarken, reinen rotstichig blauen Tönen
mit guten Echtheiten, wie insbesondere guter Lichtechtheit der trockenen oder feuchten, wie mit Trinkwasser befeuchteten, Färbungen, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit.

<u>Beispiel 2</u>

a) Zu einer Lösung von 61 Teilen 4-(γ-Aminopropylamino)-3-(β-hydroxyethylsulfonyl)-nitrobenzol in 150 Teilen N- Methyl-pyrrolidon-(2) gibt man 24 Teile Triethylamin und anschließend stetig 31 Teile Benzoylchlorid, wobei die Reaktionstemperatur auf 45°C ansteigt. Man rührt 3 Stunden nach und rührt den Ansatz sodann auf 1000 Teile Eiswasser. Nach 2 Stunden wird das in reiner Form und hoher Ausbeute angefallene 4-(γ-Benzoylaminopropylamino)-3-(β-hydroxyethylsulfonyl)-nitrobenzol abgesaugt und getrocknet; es hat einen Schmelzpunkt von 140-142°C und zeigt im 1H-NMR-Spektrum folgende Daten:
δ = 1,87 ppm (m,2H); 3,38 ppm (m,2H); 3,48 ppm (m,2H), 3,58 ppm (m,2H); 3,77 ppm (m,2H); 4,92 ppm (s,OH); 7,04 ppm (d,1H); 7,37 ppm (t,NH); 7,45 ppm (m,2H); 7,51 ppm (m,1H); 7,86 ppm (m,2H); 8,25 ppm (dd,1H); 8,45 ppm (d,1H); 8,57 ppm (t,NH).

b) 50 Teile der unter a) erhaltenen Nitroverbindungen werden in 250 Teilen Ethanol gelöst und in Gegenwart eines Nickelkatalysators in einem autoklaven bei einer Temperatur von bis zu 70°C und einem Druck von 50 bar hydriert. Die Hydrierdauer beträgt etwa vier Stunden. Der Katalysator wird sodann abfiltriert und das Filtrat durch Abdestillation des Lösemittels eingeengt. Die gewünschte Verbindung der Formel

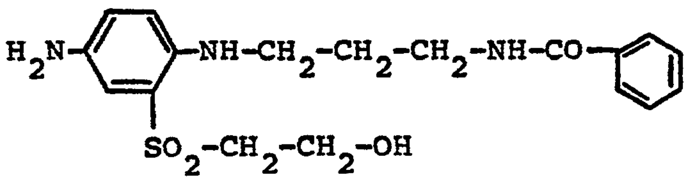

wird in form eines Öles erhalten.

c) Das aus b) erhaltene 4-(γ-Benzoylaminopropylamino)-3-(β-hydroxyethylsulfonyl)-anilin wird analog den Angaben von Beispiel 1c) und 1d) zur Triphendioxazin-Verbindung umgesetzt, die anschließend gemäß den Angaben des Beispieles 1e) zur erfindungsgemäßen Triphendioxazin-Verbindung sulfiert wird:

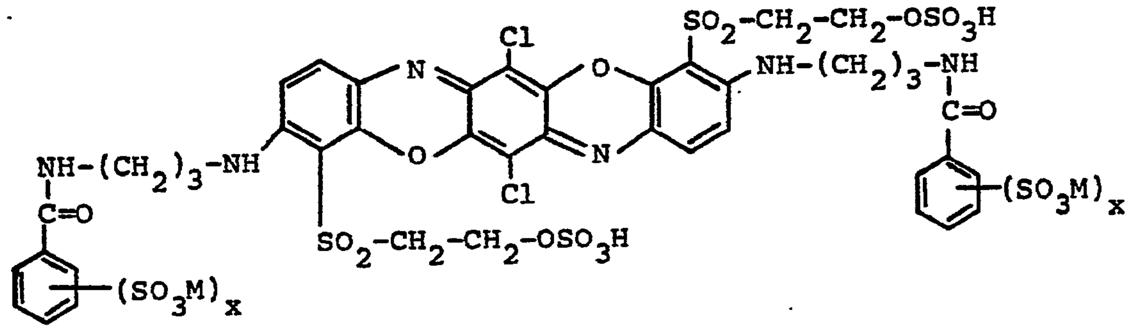

(worin x einen Sulfierungsgrad von ca. 1,2 angeben soll).

Diese erfindungsgemäße Verbindung besitzt sehr gute faserreaktive Farbstoffeigenschaften ($\lambda_{max}$ = 606 nm). Sie färbt die in der Beschreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen rotstichig blauen Tönen mit guten Echtheiten, wie insbesondere guter Lichtechtheit der trockenen oder feuchten, wie mit Trinkwasser befeuchteten, Färbungen, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit.

Beispiele 3 bis 17

In den nachfolgenden Tabellenbeispielen sind weitere erfindungsgemäße Triphendioxazin-Verbindungen mit Hilfe der Formelglieder der allgemeinen Formel (A)

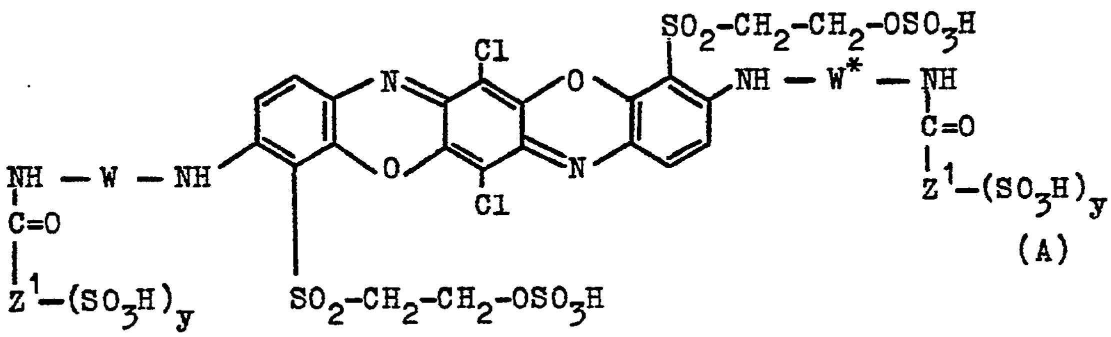

(A)

beschrieben, wobei W den in der Tabelle für W* "spiegelbildlich" geschriebenen Rest darstellt und wobei die Stellung der Sulfosubstitution in $Z^1$ unbestimmt ist, sich jedoch aus den Substitutionsregeln annähernd entnehmen ließe. Diese erfindungsgemäßen Verbindungen lassen sich in erfindungsgemäßer Weise, so beispielsweise analog den obigen Ausführungsbeispielen, aus den ersichtlichen Komponenten (Chloranil, einer Verbindung der nachstehenden allgemeinen Formel (5a) und Schwefelsäure/Oleum) herstellen.

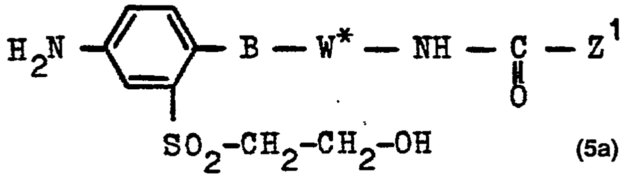

(5a)

Die erfindungsgemäßen Triphendioxazin-Verbindungen besitzen ebenfalls sehr gute faserreaktive Farbstoffeigenschaften und liefern, insbesondere auf Cellulosefasermaterialien, farbstarke Färbungen und Drucke mit guten Echtheiten in dem in dem jeweiligen Tabellenbeispiel angegebenen Farbton.

| Bsp. | Rest $-W^*-$ | Rest -Z | y | Farbton |
|---|---|---|---|---|
| 3 | $-CH_2-CH(CH_3)-$ | Phenyl | ca. 1,2 | rotstichig blau (610 nm) |
| 4 | 1,2-Ethylen | 4-Methyl-phenyl | ca. 1,1 | rotstichig blau ( 612 nm) |
| 5 | 1,3-Propylen | 4-Chlor-phenyl | ca. 1,1 | rotstichig blau |
| 6 | dito | 4-Methoxy-phenyl | ca. 1,1 | dito |
| 7 | 1,2-Ethylen | dito | ca. 1,1 | dito |
| 8 | 2-Methyl-1,3-propylen | dito | ca. 1,1 | dito |
| 9 | $-(CH_2)_2-O-(CH_2)_2-$ | dito | ca. 1,1 | dito |
| 10 | $-(CH_2)_2-SO_2-(CH_2)_2-$ | dito | ca. 1,1 | dito |
| 11 | 2-Methyl-1,3-propylen | 4-Chlor-phenyl | ca. 1,1 | dito |
| 12 | $-(CH_2)_2-N(CH_3)-(CH_2)_2-$ | dito | ca. 1,1 | dito |
| 13 | 1,2-Dimethyl-1,2-ethylen | dito | ca. 1,1 | dito |
| 14 | dito | 4-Methoxy-phenyl | ca. 1,1 | dito |
| 15 | dito | Phenyl | ca. 1,2 | dito |
| 16 | 1,2-Ethylen | 2-Methoxy-phenyl | ca. 1,2 | dito |
| 17 | 1,2-Ethylen | 1-Naphthyl | ca. 2 | dito |
| 18 | 1,2-Ethylen | 2-Methyl-phenyl | ca. 1,2 | dito |

Beispiel 19

a) 58 Teile 4-(β-Aminoethyl-amino)-3-(β-hydroxyethylsulfonyl)-nitrobenzol werden in 200 Teilen Aceton angerührt; innerhalb von 20 Minuten gibt man bei einer Temperatur zwischen 10 und 15°C langsam und stetig unter Rühren eine Lösung von 26 Teilen Phenylisocyanat in 30 Teilen Aceton hinzu und rührt anschließend bei Raumtemperatur 6 Stunden nach. Das ausgefallene Produkt wird abgesaugt und getrocknet.

Das gewünschte 4-[β-(N'-Phenyl-ureido)-ethylamino]-3-(β-hydroxyethylsulfonyl)-nitrobenzol besitzt einen Schmelzpunkt von 157 - 158°C und zeigt folgende $^1$H-NMR-Daten:

δ = 3,35 ppn (m,2H); 3,47 ppm (m,4H); 3,70 ppm (m,2H); 4,85 ppm (t,OH); 6,34 ppm (t,1H); 6,87 ppm (m,1H); 7,10 ppm (d,1H); 7,20 ppm (m,2H); 7,32 ppm (t, NH); 7,38 ppm (m,2H); 8,22 ppm (dd,1H); 8,40 ppm (d,1H); 8,56 ppm (s,NH).

b) 40 Teile der Nitroverbindung von a) werden in 200 Teilen Methanol gelöst und in Gegenwart eines Nickel-Katalysators in einem autoklaven bei einer Temperatur von bis zu 80°C und einem Druck von 50

bar hydriert. Nach beendeter Hydrierung wird der Katalysator abfiltriert und das Filtrat durch Abdampfen des Lösemittels eingeengt. Die Aminoverbindung der Formel

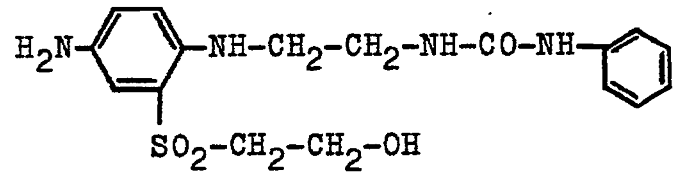

fällt in fester Form an und zeigt einen Schmelzpunkt von 139 - 143°C und folgende $^1$H-NMR-Daten: δ = 3,18 ppm (m,2H); 3,30 ppm (m,2H); 3,35 ppm (m,2H); 3,63 ppm (m,2H); 4,77 ppm (s,$NH_2$); 4,85 ppm (s,OH); 5,48 ppm (t,NH); 6,28 ppm (t,NH); 6,77 ppm (d,1H); 6,85 ppm (dd,1H); 6,90 ppm (t,1H); 6,94 ppm (d,1H); 7,22 ppm (t,2H); 7,40 ppm (d,2H); 8,50 ppm (s,NH).

c) 15,3 Teile der Anilinverbindung von b), 0,8 Teile Magnesiumoxid und 5,2 Teile Chloranil werden zusammen in 150 Teilen Ethanol während 5 Stunden bei 40°C erwärmt. Das entstandene, ausgefallene Produkt wird abgesaugt, der Rückstand in 150 Teilen Wasser angerührt, wieder abgesaugt und bei reduziertem Druck bei 80°C getrocknet.

9,3 Teile dieses Kondensationsproduktes werden in 95 Teile 10 %igem Oleum innerhalb von 15 Minuten bei 20°C unter Rühren eingetragen; man rührt noch bis zur vollständigen Lösung des Produktes nach. Anschließend werden 4,7 Teile Natriumperoxodisulfat langsam eingetragen, so daß die Temperatur des Ansatzes 25°C nicht Überschreitet. Es wird noch eine Stunde nachgerührt, der Ansatz sodann auf Eis gegeben, diese Suspension mit Calciumcarbonat auf einen pH-Wert zwischen 1 und 1,5 gestellt und anschließend mit Natriumcarbonat bis zur Einstellung eines pH-Wertes von 5,5 versetzt; das Calciumsulfat wird abgesaugt, mit Wasser gewaschen, und die Filtrate werden vereinigt. Die erfindungsgemäße Verbindung kann aus dem fitlrat durch Eindampfen oder Sprühtrocknung isoliert werden.

Es wird das Natriumsalz der Verbindung der Formel

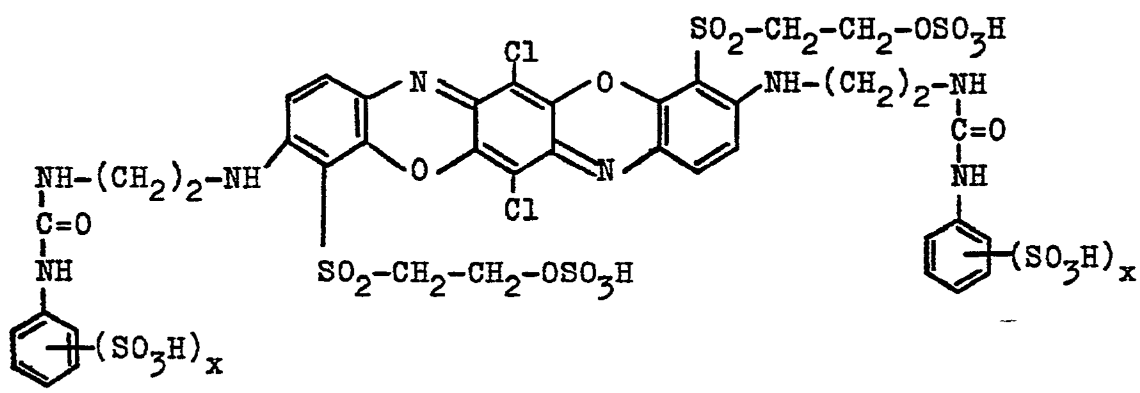

(worin x einen Sulfierungsgrad von ca. 1,2 angeben soll) als elektrolytsalzhaltiges (vorwiegend Natriumsulfathaltiges) Pulver (die jeweilige β-Sulfatoethylsulfonyl-Gruppe kann auch in der anderen ortho-Stellung zur Aminogruppe gebunden sein, befindet sich jedoch mit größerer Wahrscheinlichkeit in der in der obigen Formel angegebenen Stellung).

Diese erfindungsgemäße Verbindung besitzt sehr gute faserreaktive Farbstoffeigenschaften ($\lambda_{max}$ = 623 nm. Sie färbt die in der Beschreibung genannten Materialien, insbesondere Celulosefasermaterialien, wie Baumwolle, nach den in der Technik üblichen und bekannten Verfahrensweisen der Applikation und Fixierung von faserreaktiven Farbstoffen in farbstarken, reinen blauen Tönen mit guten Echtheiten, wie insbesondere guter Lichtechtheit der trockenen oder feuchten, wie mit Trinkwasser befeuchteten, Färbungen, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit.

Beispiele 20 bis 37

In den nachfolgenden Tabellenbeispielen sind weitere erfindungsgemäße Triphendioxazin-Verbindungen mit Hilfe der Formelglieder der allgemeinen Formel (B)

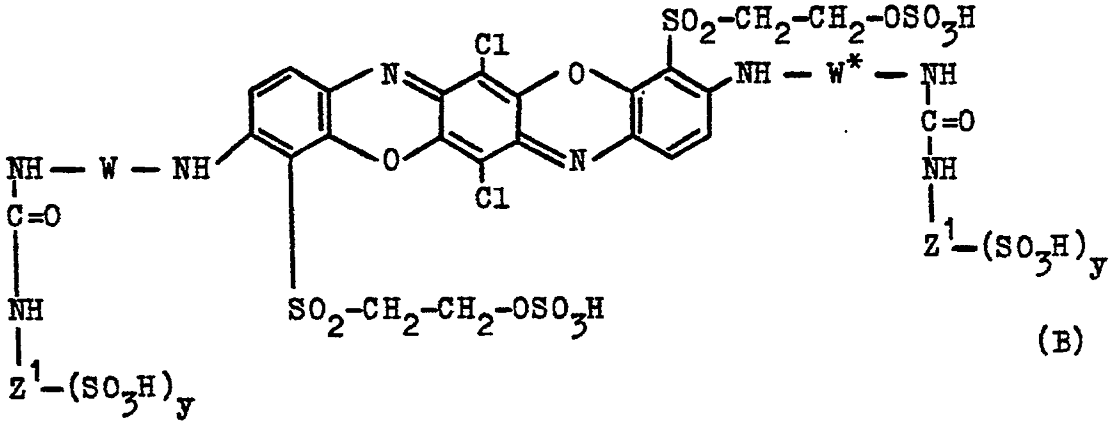

(B)

beschrieben, wobei W den in derTabelle für W* "spiegelbildlich" geschriebenen Rest darstellt und wobei die Stellung der Sulfosubstitution in $Z^1$ unbestimmt ist, sich jedoch aus den Substitutionsregeln annähernd entnehmen ließe. Diese erfindungsgemäßen Verbindungen lassen sich in erfindungsgemäßer Weise, so beispielsweise analog den obigen Ausführungsbeispielen, aus den ersichtlichen Komponenten (Chloranil, einer Verbindung der nachstehenden allgemeinen Formel (5b) und Schwefelsäure/Oleum) herstellen.

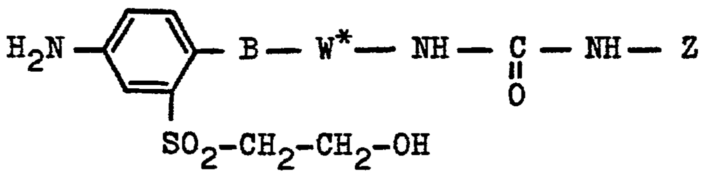

(5b)

Diese erfindungsgemäßen Triphendioxazin-Verbindungen besitzen ebenfalls sehr gute faserreaktive Farbstoffeigenshaften und liefern insbesondere auf Cellulosefasermaterialien farbstarke Färbungen und Drucke mit guten Echtheiten in dem in dem jeweiligen Tabellenbeispiel angegebenen Farbton.

| Bsp. | Rest -W*- | Rest -Z | y | Farbton |
|---|---|---|---|---|
| 20 | 1,3-Propylen | Phenyl | ca. 1,2 | blau (615 nm) |
| 21 | $-CH_2-CH(CH_3)-$ | Phenyl | ca. 1,2 | blau (620 nm) |
| 22 | $-(CH_2)_2-O-(CH_2)_2-$ | Phenyl | ca. 1,2 | blau |
| 23 | 1,2-Ethylen | 3-Methyl-phenyl | ca. 1,1 | blau |
| 24 | 1,3-Propylen | dito | ca. 1,1 | blau |
| 25 | $-CH_2-CH(CH_3)-$ | dito | ca. 1,1 | blau |
| 26 | 1,2-Ethylen | 2-Methyl-phenyl | ca. 1,1 | blau |
| 27 | 1,3-Propylen | dito | ca. 1,1 | blau |
| 28 | $-(CH_2)_2-SO_2-(CH_2)_2-$ | Phenyl | ca. 1,2 | blau |
| 29 | dito | 3-Methyl-phenyl | ca. 1,1 | blau |
| 30 | 1,2-Ethylen | 2-Chlor-phenyl | ca. 1,2 | blau |
| 31 | 1,3-Propylen | 2,3-Dichlor-phenyl | 1 | blau |
| 32 | 1,2-Ethylen | 3,5-Dimethyl-phenyl | 1 | blau |
| 33 | 2-Methyl-1,3-propylen | dito | 1 | blau |
| 34 | 1,2-Ethylen | 1-Naphthyl | ca. 2 | blau |
| 35 | 1,3-Propylen | dito | ca. 2 | blau |
| 36 | 1,2-Ethylen | 4-Chlor-phenyl | ca. 1,2 | blau |
| 37 | 1,3-Propylen | dito | ca. 1,2 | blau |

**Patentansprüche für die Vertragsstaaten: BE, CH, DE, FR, GB, IT, LI**

1. Eine wasserlösliche Triphendioxazin-Verbindung, die der allgemeinen Formel (1)

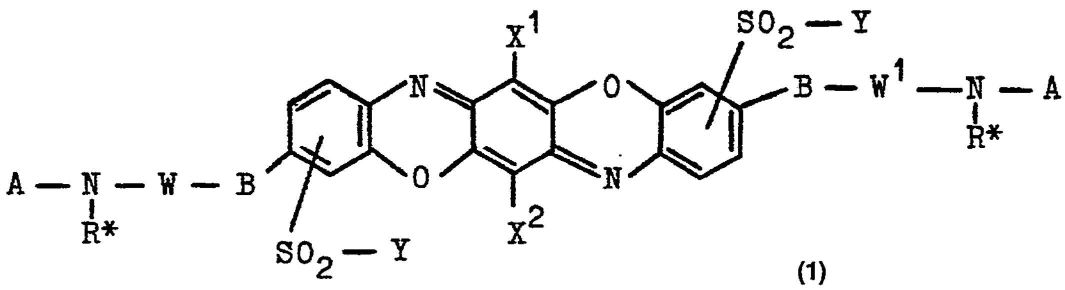

(1)

entspricht, worin bedeuten:

B ist ein Sauerstoff- oder Schwefelatom oder eine Aminogruppe der Formel -NH- oder -N(R′)-, in welcher
R′ eine Alkylgruppe von 1 bis 6 C-Atomen ist;
R* ist ein Wasserstoffatom oder eine gegebenenfalls substituierte Alkylgruppe von 1 bis 4 C-Atomen oder ein gegebenenfalls substituierter Arylrest;
W ist ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest, wobei die aliphatischen Reste durch Heterogruppen unterbrochen sein können, die aus den Gruppen -O- , -S- , $-SO_2-$ , -CO- , 1,4-Piperidino, -NH- und $-N(R^0)-$ , worin $R^0$ eine der Bedeutungen von R′ hat oder eine Alkanoylgruppe von 2 bis 5 C-Atomen ist, ausgewählt sind, und
$W^1$ hat eine der für W angegebenen Bedeutungen und ist mit W gleich oder von W verschieden, oder
die Gruppierung $-B-W^1-N(R^*)-$ und die Gruppierung -N(R*)-W-B- , zueinander gleich oder voneinander verschieden, stellt jede gemeinsam den bivalenten Rest eines fünf- oder sechsgliedrigen, zwei Stickstoffatome enthaltenden gesättigten Heterocyclus dar oder
die Gruppierung $-B-W^1-$ und die Gruppierung -W-B- , zueinander gleich oder voneinander verschieden, stellt jede gemeinsam den bivalenten Rest eines fünf- oder sechsgliedrigen, zwei Stickstoffatome enthaltenden gesättigten Heterocyclus dar, der mit einem der beiden Stickstoffatome über eine Alkylengruppe von 2 bis 4 C-Atomen mit der Gruppierung -N(R*)-A bzw. A-N(R*)- verbunden ist;
A ist ein Rest der Formel (2a) oder (2b)

(2a) 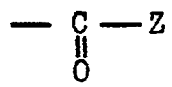

(2b) 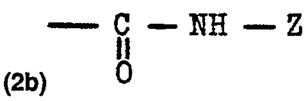

in welchen
Z ein Phenylrest oder ein Naphthylrest ist, die beide substituiert sind, wobei mindestens einer dieser Substituenten einen wasserlöslich-machende Gruppe ist;
$X^1$ ist ein Wasserstoffatom, ein Halogenatom, eine Alkylgruppe von 1 bis 4 C-Atomen, eine Alkoxygruppe von 1 bis 4 C-Atomen oder ein gegebenenfalls substituierter Arylrest;
$X^2$ ist mit $X^1$ gleich oder von $X^1$ verschieden und hat eine der für $X^1$ angegebenen Bedeutungen;
Y ist die Vinylgruppe oder eine Ethylgruppe, die in β-Stellung einen durch ein Alkali eliminierbaren Substituenten enthält;
die Gruppe $-SO_2-Y$ steht bevorzugt in ortho-Stellung zur Gruppe -B-W-N(R*)-A bzw. A-N(R*)-W-B- gebunden;
von den Sulfogruppen, die im Molekül (1) enthalten sein können, enthält das Molekül (1) mindestens zwei.

2. Verbindung nach Anspruch 1, dadurch gekennzeichnet, daß Z ein Phenylrest ist, der durch Alkyl von 1 bis 4 C-Atomen, Alkoxy von 1 bis 4 C-Atomen, Chlor, Brom, Nitro, Carbalkoxy von 2 bis 5 C-Atomen, Sulfo und/oder Carboxy substituiert ist, wobei mindestens einer dieser Substituenten eine Sulfo- oder Carboxygruppe ist.

3. Verbindung nach Anspruch 1, dadurch gekennzeichnet, daß Z ein Rest der allgemeinen Formel (3a) oder (3b)

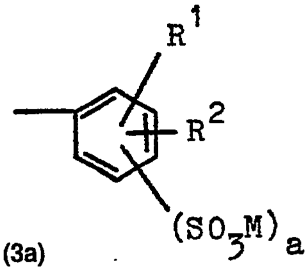

(3a)

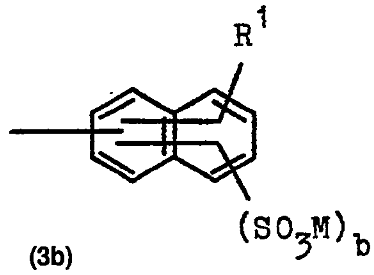

(3b)

ist, in welchen
M ein Wasserstoffatom oder ein Alkalimetall oder das Äquivalent eines Erdalkalimetalls ist,
$R^1$ ein Wasserstoffatom, eine Alkylgruppe von 1 bis 4 C-Atomen, eine Alkoxygruppe von 1 bis 4 C-Atomen, ein Chloratom, ein Bromatom, eine Nitrogruppe, eine Carbalkoxygruppe von 2 bis 5 C-Atomen oder eine Sulfogruppe ist,
$R^2$ ein Wasserstoffatom, eine Alkylgruppe von 1 bis 4 C-Atomen, eine Alkoxygruppe von 1 bis 4 C-Atomen, ein Chloratom, eine Carboxygruppe oder eine Sulfogruppe ist,
a für die Zahl, Null oder 1 steht, wobei a zwingend 1 ist, falls $R^1$ oder $R^2$ keine der angegebenen wasserlöslich-machenden Gruppen bedeutet, und
b die Zahl Null, 1 oder 2 bedeutet, wobei b zwingend die Zahl 1 oder 2 ist, falls $R^1$ keine der genannten wasserlöslich-manchenden Gruppen darstellt.

4. Verbindung nach Anspruch 3, dadurch gekennzeichnet, daß in Formel (3a) $R^1$ ein Wasserstoffatom, die Methyl-, Ethyl-, Methoxy-, Ethoxy- oder Sulfogruppe oder ein Chlor- oder Bromatom ist und $R^2$ ein Wasserstoffatom, die Methoxy-, Ethoxy-, Carboxy- oder Sulfogruppe oder ein Chloratom bedeutet sowie a für die Zahl 1 steht.

5. Verbindung nach Anspruch 3, dadurch gekennzeichnet, daß Z ein Rest der Formel (3b) ist, in welcher $R^1$ ein Wasserstoffatom bedeutet und b die Zahl 1 oder 2 ist.

6. Verbindung nach mindestens einem der Ansprüche 1 bis 5, dadurch gekennzeichnet, daß W und $W^1$ beide, zueinander gleich oder voneinander verschieden, einen Alkylenrest von 2 bis 6 C-Atomen bedeuten, der durch ein Sauerstoffatom oder eine Gruppe -NH- substituiert sein kann, oder jeder eine Cycloalkylengruppe von 5 oder 6 C-Atomen mit gegebenenfalls 1, 2 oder 3 Methylgruppen als Substituenten oder zwei solche mit einem Alkylenrest von 1 bis 4 C-Atomen verbundene Cycloalkylengruppen oder einen Alkylenrest von 2 bis 6 C-Atomen, der durch eine solche Cylcoalkylengruppe unterbrochen ist, darstellen.

7. Verbindung nach mindestens einem der Ansprüche 1 bis 5, dadurch gekennzeichnet, daß W und $W^1$ beide für den 1,2-Ethylen-, 1,3-Propylen-, 1,4-Butylen- oder den Isopropylen-Rest stehen.

8. Verbindung nach mindestens einem der Ansprüche 1 bis 7, dadurch gekennzeichnet, daß beide Y eine β-Sulfatoethyl-Gruppe bedeuten.

9. Verbindung nach mindestens einem der Ansprüche 1 bis 8, dadurch gekennzeichnet, daß $X^1$ und $X^2$ beide ein Chlor- oder ein Bromatom, bevorzugt ein Chloratom, bedeuten.

10. Verbindung nach Anspruch 1 der allgemeinen Formel (1a)

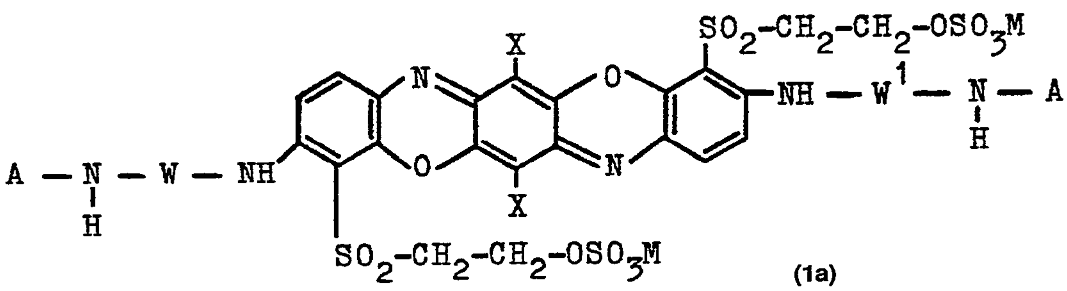

(1a)

in welcher
W und $W^1$ beide eine Alkylengruppe von 2 bis 6 C-Atomen bedeuten,
X jedes für ein Bromatom oder insbesondere bevorzugt ein Chloratom steht,
M ist ein Wasserstoffatom oder ein Alkalimetall und
A beide eine Gruppe der in Anspruch 1 genannten Formel (2a) oder (2b) bedeuten, in welchen die Gruppe Z, jeweils zueinander gleich oder voneinander verschieden, eine Gruppe der allgemeinen Formel (3c)

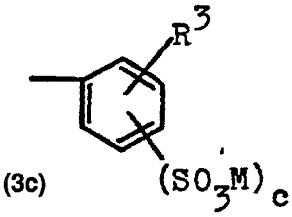

(3c)

ist, in welcher
$R^3$ die Methyl-, Ethyl-, Methoxy- oder Ethoxygruppe oder eine Sulfogruppe oder ein Chlor- oder Bromatom bedeutet, und
c für die Zahl Null, 1 oder 2 steht, wobei c zwingend die Zahl 1 oder 2 ist, falls $R^3$ keine Sulfogruppe bedeutet.

11. Verfahren zur Herstellung einer in Anspruch 1 genannten und definierten Verbindung der allgemeinen Formel (1), dadurch gekennzeichnet, daß man eine Verbindung der allgemeinen Formel (4)

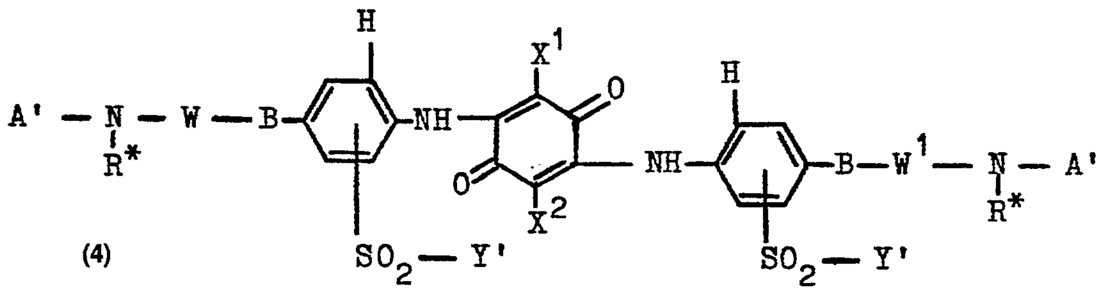

(4)

(in welcher Y′ die Vinylgruppe, die β-Hydroxyethyl-Gruppe oder eine Ethylgruppe, die in β-Stellung einen durch ein Alkali eliminierbaren Substituenten, wie einen der für Y genannten, enthält, bevorzugt die β-Hydroxyethyl-Gruppe ist, A′ die Bedeutung von A hat, wobei die Gruppen Z jedoch nicht zwingend eine wasserlöslich-machende Gruppe enthalten müssen, und B, R*, W, $W^1$, $X^1$ und $X^2$ die in Anspruch 1 genannten Bedeutungen haben, und wobei substituierte Alkylgruppen in diesen Resten auch hydroxysubstituierte Alkylgruppen sein können, die Gruppen $-SO_2-Y'$ bevorzugt in ortho-Stellung zur Gruppe B gebunden sind und die Benzolkerne in einer der ortho-Stellungen zur angegebenen Aminogruppe -NH- nicht substituiert sein dürfen) in saurem Medium und vorzugsweise in Gegenwart eines Oxidationsmittels zum Triphendioxazin cyclisiert und gegebenenfalls während der Cyclisierung oder nach der Cyclisierung eine oder mehrere Sulfogruppen in einem Arylrest einführt.

12. Verfahren nach Anspruch 11, dadurch gekennzeichnet, daß man gleichzeitig mit der Cyclisierung oder erst nach der Cyclisierung gegebenenfalls vorhandene Hydroxyalkylgruppen in Sulfatoalkyl- oder Phosphatoalkylgruppen überführt,
oder daß man gleichzeitig mit der Cyclisierung mittels Schwefelsäure oder Schwefeltrioxid enthaltender Schwefelsäure Hydroxyalkylgruppen in Sulfatoalkylgruppen überführt und Arylrest sulfiert,
oder
daß man die Sulfierungsreaktion nach der Cyclisierung bei einer Temperatur zwischen 20 und 80°C mittels Schwefelsäure oder Schwefeltrioxid enthaltender Schwefelsäure durchführt,
oder daß
man die Cyclisierung in Schwefelsäure oder Schwefeltrioxid enthaltender Schwefelsäure mit einem Alkalipersulfat durchführt.

13. Verwendung einer in Anspruch 1 genannten und definierten Verbindung der allgemeinen Formel (1) zum Färben (einschließlich Bedrucken) von hydroxy- und/oder carbonamidgruppenhaltigem Material, insbesondere Fasermaterial.

14. Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder in das Material einbringt und ihn mittels Wärme und/oder mit Hilfe eines alkalisch wirkenden Mittels fixiert, dadurch gekennzeichnet, daß man als Farbstoff eine Verbindung der in Anspruch 1 genannten und definierten allgemeinen Formel (1) einsetzt.

15. Eine Verbindung der allgemeinen Formel (12)

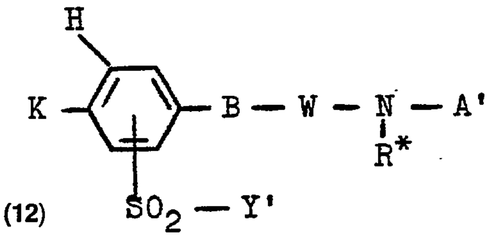
(12)

in welcher
K die Nitrogruppe oder die Aminogruppe ist,
B ein Sauerstoff- oder Schwefelatom oder eine Aminogruppe der Formel -NH- oder -N(R')- ist, in welcher
R' eine Alkylgruppe von 1 bis 5 C-Atomen bedeutet,
B hiervon bevorzugt die Guppe -NH- ist,
W ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest ist, wobei die aliphatischen Reste durch Heterogruppen unterbrochen sein können, die aus den Gruppen -O- , -S- , $-SO_2-$ , -CO- , 1,4-Piperidino, -NH- und -N($R^0$)- , worin $R^0$ eine der Bedeutungen von R' hat oder eine Alkanoylgruppe von 2 bis 5 C-Atomen ist, ausgewählt sind,
R* ein Wasserstoffatom oder ein gegebenenfalls substituierte Alkylgruppe von 1 bis 4 C-Atomen oder eingegebenenfalls substituierter Arylrest ist,
Y' die Vinylgruppe oder eine Ethylgruppe bedeutet, die in β-Stellung einen durch ein Alkali eliminierbaren Substituenten enthält, oder die β-Hydroxyethyl-Gruppe ist und
A' für einen Rest der allgemeinen Formel (2c) oder (2d)

(2c) 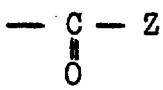 (2d) 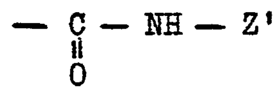

steht, in welchen
Z' ein Phenylrest oder ein Naphthylrest ist, die beide substituiert sein können, bevorzugt ein Rest der Formel (3d), (3e) oder (3f)

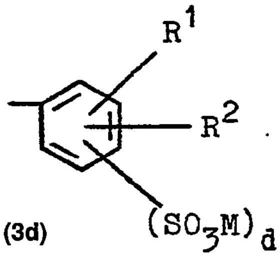
(3d)

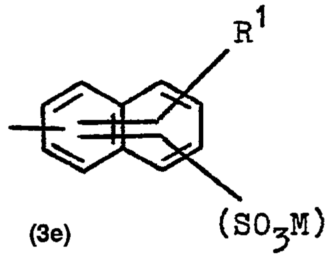
(3e)

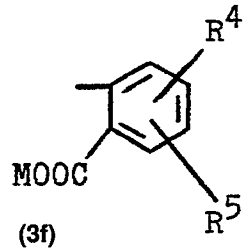
(3f)

ist, in welchen
$R^1$, $R^2$ und M die in Anspruch 3 genannten Bedeutungen haben,
d für die Zahl Null, 1 oder 2 steht,
e die Zahl Null, 1, 2 oder 3 bedeutet,
$R^4$ ein Wasserstoffatom, eine Alkylgruppe von 1 bis 4 C-Atomen, eine Alkoxygruppe von 1 bis 4 C-Atomen, ein Chloratom, ein Bromatom oder eine Nitrogruppe und
$R^5$ ein Wasserstoffatom, eine Alkylgruppe von 1 bis 4 C-Atomen, eine Alkoxygruppe von 1 bis 4 C-Atomen oder ein Choratom ist.

16. Verfahren zur Herstellung einer in Anspruch 15 angegebenen und definierten Verbindung der allgemeinen Formel (12), dadurch gekennzeichnet, daß man eine Verbindung der allgemeinen Formel (7)

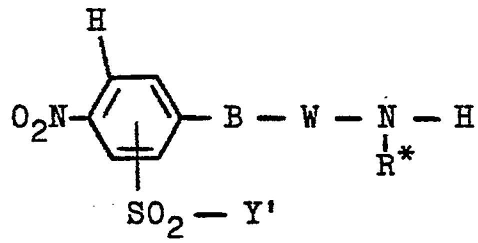

(7)

in welcher Y', B, W und R* die in Anspruch 15 genannten Bedeutungen haben, mit einer Verbindung der allgemeinen Formel (8A), (8B) oder (8C)

Cl — CO — Z'

(8A)

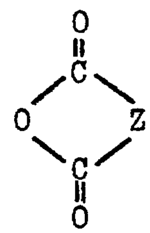

(8B)

O=C=N — Z'

(8C)

mit Z' der in Anspruch 15 angegebenen Bedeutung, bevorzugt mit einer Verbindung der allgemeinen Formel (8a), (8b), (8c), (9a) oder (9b)

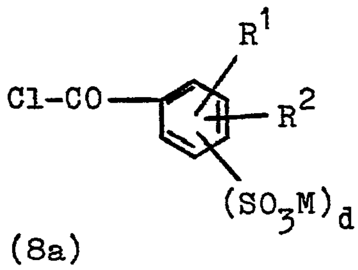

(8a)

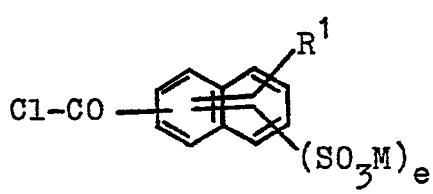

(8b)

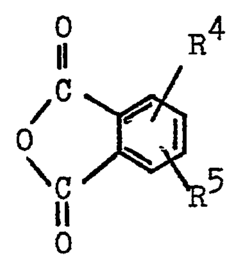

(8c)

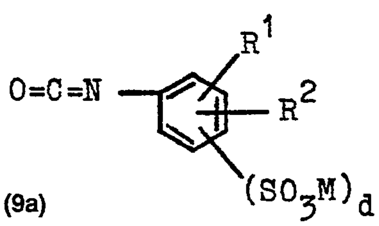

(9a)

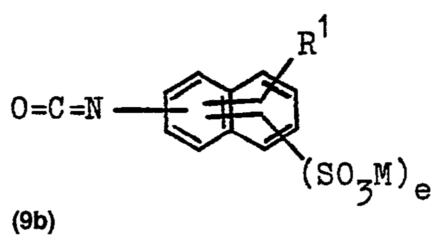

(9b)

in welchen $R^1$, $R^2$, $R^3$, $R^4$, $R^5$, M, d und e die in Anspruch 15 genannten Bedeutungen haben, umsetzt und gegebenenfalls in der so erhaltenen Verbindung der allgemeinen Formel (12) mit K der Nitrogruppe die Nitrogruppe zur Aminogruppe reduziert.

17. Verwendung einer Verbindung der allgemeinen Formel (12) von Anspruch 15 zur Synthese von Farbstoffen, insbesondere von Triphenydioxazin-Verbindungen der allgemeinen Formel (1) gemäß Anspruch 1.

**Patentansprüche für den Vertragsstaat : ES**

1. Verfahren zur Herstellung einer wasserlöslichen Triphendioxazin-Verbindung, die der allgemeinen Formel (1)

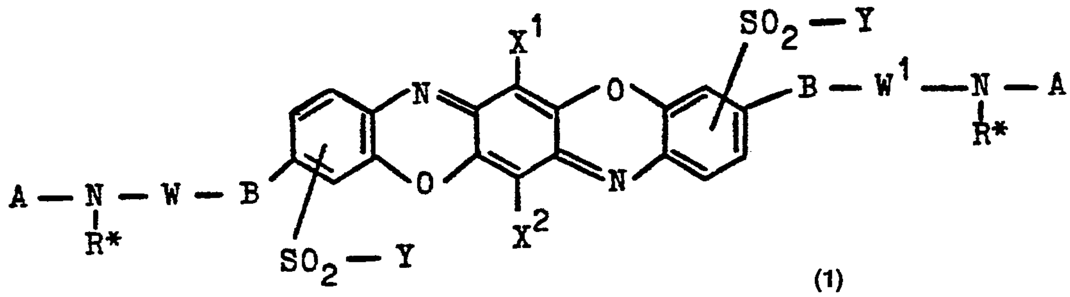

(1)

entspricht, worin bedeuten:

B ist ein Sauerstoff- oder Schwefelatom oder eine Aminogruppe der Formel -NH- oder -N(R')-, in welcher
R' eine Alkylgruppe von 1 bis 6 C-Atomen ist;
R* ist ein Wasserstoffatom oder eine gegebenenfalls substituierte Alkylgruppe von 1 bis 4 C-Atomen oder ein gegebenenfalls substituierter Arylrest;
W ist ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest, wobei die aliphatischen Reste durch Heterogruppen unterbrochen sein können, die aus den Gruppen -O- , -S- , $-SO_2-$ , -CO- , 1,4-Piperidino, -NH- und $-N(R^0)-$ , worin $R^0$ eine der Bedeutungen von R' hat oder eine Alkanoylgruppe von 2 bis 5 C-Atomen ist, ausgewählt sind, und
$W^1$ hat eine der für W angegebenen Bedeutungen und ist mit W gleich oder von W verschieden, oder
die Gruppierung $-B-W^1-N(R^*)-$ und die Gruppierung -N(R*)-W-B- , zueinander gleich oder voneinander verschieden, stellt jede gemeinsam den bivalenten Rest eines fünf- oder sechsgliedrigen, zwei Stickstoffatome enthaltenden gesättigten Heterocyclus dar oder
die Gruppierung $-B-W^1-$ und die Gruppierung -W-B- , zueinander gleich oder voneinander verschieden, stellt jede gemeinsam den bivalenten Rest eines fünf- oder sechsgliedrigen, zwei Stickstoffatome enthaltenden gesättigten Heterocyclus dar, der mit einem der beiden Stickstoffatome über eine Alkylengruppe von 2 bis 4 C-Atomen mit der Gruppierung -N(R*)-A bzw. A-N(R*)- verbunden ist;
A ist ein Rest der Formel (2a) oder (2b)

(2a) 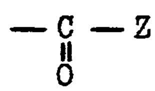 (2b) 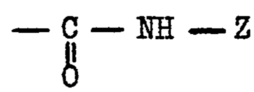

in welchen
Z ein Phenylrest oder ein Naphthylrest ist, die beide substituiert sind, wobei mindestens einer dieser Substituenten einen wasserlöslich-machende Gruppe ist;
$X^1$ ist ein Wasserstoffatom, ein Halogenatom, eine Alkylgruppe von 1 bis 4 C-Atomen, eine Alkoxygruppe von 1 bis 4 C-Atomen oder ein gegebenenfalls substituierter Arylrest;
$X^2$ ist mit $X^1$ gleich oder von $X^1$ verschieden und hat eine der für $X^1$ angegebenen Bedeutungen;
Y ist die Vinylgruppe oder eine Ethylgruppe, die in β-Stellung einen durch ein Alkali eliminierbaren Substituenten enthält;
die Gruppe $-SO_2$-Y steht bevorzugt in ortho-Stellung zur Gruppe -B-W-N(R*)-A bzw. A-N(R*)-W-B- gebunden;
von den Sulfogruppen, die im Molekül (1) enthalten sein können, enthält das Molekül (1) mindestens zwei, dadurch gekennzeichnet, daß man eine Verbindung der allgemeinen Formel (4)

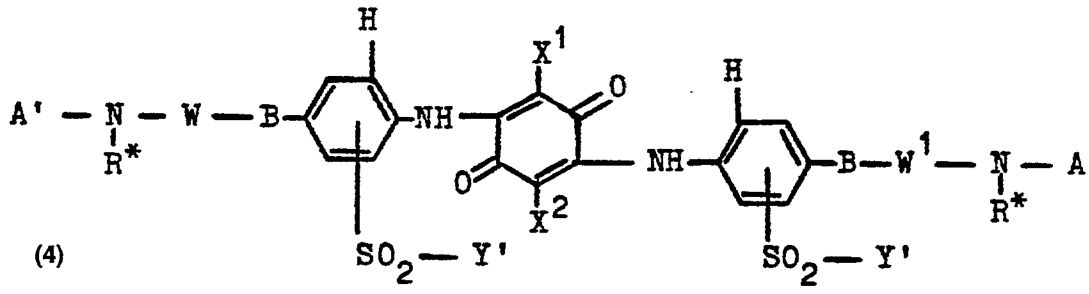

(4)

(in welcher Y′ die Vinylgruppe, die β-Hydroxyethyl-Gruppe oder eine Ethylgruppe, die in β-Stellung einen durch ein Alkali eliminierbaren Substituenten, wie einen der für Y genannten, enthält, bevorzugt die β-Hydroxyethyl-Gruppe ist, A′ die Bedeutung von A hat, wobei die Gruppen Z jedoch nicht zwingend eine wasserlöslich-machende Gruppe enthalten müssen, und B, R*, W, $W^1$, $X^1$ und $X^2$ die oben genannten Bedeutungen haben, und wobei substituierte Alkylgruppen in diesen Resten auch hydroxysubstituierte Alkylgruppen sein können, die Gruppen $-SO_2-Y'$ bevorzugt in ortho-Stellung zur Gruppe B gebunden sind und die Benzolkerne in einer der ortho-Stellungen zur angegebenen Aminogruppe -NH- nicht substituiert sein dürfen) in saurem Medium und vorzugweise in Gegenwart eines Oxidationsmittels zum Triphendioxazin cyclisiert und gegebenenfalls während der Cyclisierung oder nach der Cyclisierung eine oder mehrere Sulfogruppen in einen Arylrest einführt.

2. Verfahren nach Anspruch 1, dadurch gekennzeichnet, daß Z ein Phenylrest ist, der durch Alkyl von 1 bis 4 C-Atomen, Alkoxy von 1 bis 4 C-Atomen, Chlor, Brom, Nitro, Carbalkoxy von 2 bis 5 C-Atomen, Sulfo und/oder Carboxy substituiert ist, wobei mindestens einer dieser Substituenten eine Sulfo- oder Carboxygruppe ist.

3. Verfahren nach Anspruch 1, dadurch gekennzeichnet, daß Z ein Rest der allgemeinen Formel (3a) oder (3b)

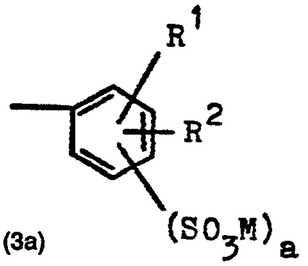

(3a)

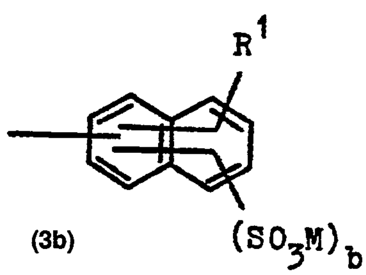

(3b)

ist, in welchen

M ein Wasserstoffatom oder ein Alkalimetall oder das Äquivalent eines Erdalkalimetalls ist,

$R^1$ ein Wasserstoffatom, eine Alkylgruppe von 1 bis 4 C-Atomen, eine Alkoxygruppe von 1 bis 4 C-Atomen ein Chloratom, ein Bromatom, eine Nitrogruppe, eine Carbalkoxygruppe von 2 bis 5 C-Atomen oder eine Sulfogruppe ist,

$R^2$ ein Wasserstoffatom, eine Alkylgruppe von 1 bis 4 C-Atomen, eine Alkoxygruppe von 1 bis 4 C-Atomen, ein Chloratom, eine Carboxygruppe oder eine Sulfogruppe ist,

a für die Zahl Null oder 1 steht, wobei a zwingend 1 ist, falls $R^1$ oder $R^2$ keine der angegebenen wasserlöslich-machenden Gruppen bedeutet, und

b die Zahl Null, 1 oder 2 bedeutet, wobei b zwingend die Zahl 1 oder 2 ist, falls $R^1$ keine der genannten wasserlöslich-machenden Gruppen darstellt.

4. Verfahren nach Anspruch 3, dadurch gekennzeichnet, daß in Formel (3a) $R^1$ ein Wasserstoffatom, die Methyl-, Ethyl-, Methoxy-, Ethoxy- oder Sulfogruppe oder ein Chlor- oder Bromatom ist und $R^2$ ein Wasserstoffatom, die Methoxy-, Ethoxy-, Carboxy- oder Sulfogruppe oder ein Chloratom bedeutet sowie a für die Zahl 1 steht.

5. Verfahren nach Anspruch 3, dadurch gekennzeichnet, daß Z ein Rest der Formel (3b) ist, in welcher $R^1$ ein Wasserstoffatom bedeutet und b die Zahl 1 oder 2 ist.

6. Verfahren nach mindestens einem der Ansprüche 1 bis 5, dadurch gekennzeichnet, daß W und $W^1$ beide, zueinander gleich oder voneinander verschieden, einen Alkylenrest von 2 bis 6 C-Atomen bedeuten, der durch ein Sauerstoffatom oder eine Gruppe -NH- unterbrochen sein kann, oder jeder eine Cycloalkylengruppe von 5 oder 6 C-Atomen mit gegebenenfalls 1, 2 oder 3 Methylgruppen als Substituenten oder zwei solche mit einem Alkylenrest von 1 bis 4 C-Atomen verbundene Cycloalkylengruppen oder einen Alkylenrest von 2 bis 6 C-Atomen, der durch eine solche Cylcoalkylengruppe unterbrochen ist, darstellen.

7. Verfahren nach mindestens einem der Ansprüche 1 bis 5, dadurch gekennzeichnet, daß W und W[1] beide für den 1,2-Ethylen-, 1,3-Propylen-, 1,4-Butylen- oder den Isopropylen-Rest stehen.

8. Verfahren nach mindestens einem der Ansprüche 1 bis 7, dadurch gekennzeichnet, daß beide Y eine β-Sulfatoethyl-Gruppe bedeuten.

9. Verfahren nach mindestens einem der Ansprüche 1 bis 8, dadurch gekennzeichnet, daß $X^1$ und $X^2$ beide ein Chlor- oder ein Bromatom, bevorzugt ein Chloratom, bedeuten.

10. Verfahren nach Anspruch 1, dadurch gekennzeichnet, daß die Verbindung der allgemeinen Formel (1) eine Verbindung der allgemeinen Formel (1a)

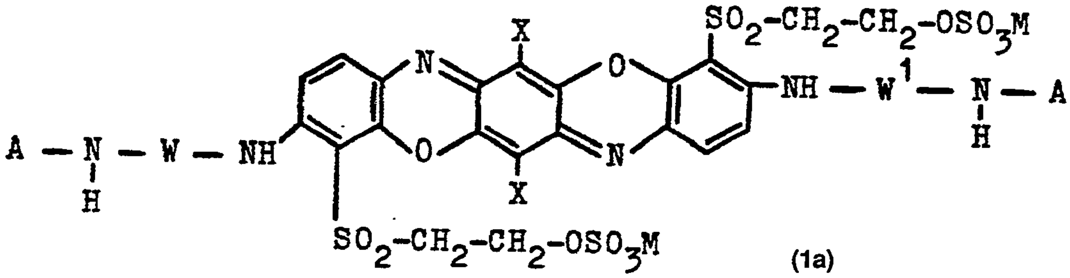

(1a)

ist, in welcher
W und W[1] beide eine Alkylengruppe von 2 bis 6 C-Atomen bedeuten,
X jedes für ein Bromatom oder insbesondere bevorzugt ein Chloratom steht,
M ist ein Wasserstoffatom oder ein Alkalimetall und
A beide eine Gruppe der in Anspruch 1 genannten Formel (2a) oder (2b) bedeuten, in welchen die Gruppe Z, jeweils zueinander gleich oder voneinander verschieden, eine Gruppe der allgemeinen Formel (3c)

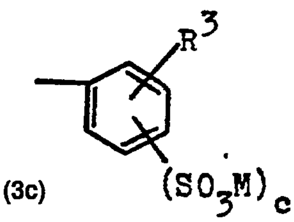

(3c)

ist, in welcher
$R^3$ die Methyl-, Ethyl-, Methoxy- oder Ethoxygruppe oder eine Sulfogruppe oder ein Chlor- oder Bromatom bedeutet, und
c für die Zahl Null, 1 oder 2 steht, wobei c zwingend die Zahl 1 oder 2 ist, falls $R^3$ keine Sulfogruppe bedeutet.

11. Verfahren nach Anspruch 1, dadurch gekennzeichnet, daß man gleichzeitig mit der Cyclisierung oder erst nach der Cyclisierung gegebenenfalls vorhandene Hydroxyalkylgruppen in Sulfatoalkyl- oder Phosphatoalkyl gruppen überführt,
oder daß man gleichzeitig mit der Cyclisierung mittels Schwefelsäure oder Schwefeltrioxid enthaltender Schwefelsäure Hydroxyalkylgruppen in Sulfatoalkylgruppen überführt und Arylreste sulfiert,
oder daß
man die Sulfierungsreaktion nach der Cyclisierung bei einer Temperatur zwischen 20 und 80°C mittels Schwefelsäure durchführt,
oder daß
man die Cyclisierung in Schwefelsäure oder Schwefeltrioxid enthaltender Schwefelsäure mit einem Alkalipersulfat durchführt.

12. Verwendung einer in Anspruch 1 genannten und definierten oder einer nach Anspruch 1 oder 11 hergestellten Verbindung der allgemeinen Formel (1) zum Färben (einschließlich) Bedrucken) von hydroxy- und/oder carbonamidgruppenhaltigem Material, insbesondere Fasermaterial.

13. Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder in das Material einbringt und ihn mittels Wärme und/oder mit Hilfe eines Alkalisch wirkenden Mittels fixiert, dadurch gekennnzeichnet, daß man als Farbstoff einen Verbindung der in Anspruch 1 genannten und definierten allgemeinen Formel (1) oder einer nach Anspruch 1 oder 11 hergestellten Verbindung der Formel (1) einsetzt.

14. Verfahren zur Herstellung einer Verbindung der allgemeinen Formel (12)

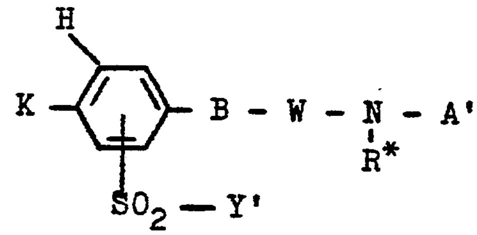

(12)

in welcher
K die Nitrogruppe oder die Aminogruppe ist,
B ein Sauerstoff- oder Schwefelatom oder eine Aminogruppe der Formel -NH- oder -N(R')- ist, in welcher
R' eine Alkylgruppe von 1 bis 5 C-Atomen bedeutet,
B hiervon bevorzugt die Guppe -NH- ist,
W ein bivalenter, aliphatischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest ist, wobei die aliphatischen Reste durch Heterogruppen unterbrochen sein können, die aus den Gruppen -O- , -S- , $-SO_2-$ , -CO- , 1,4-Piperidino, -NH- und -N($R^0$)- , worin $R^0$ eine der Bedeutungen von R' hat oder eine Alkanoylgruppe von 2 bis 5 C-Atomen ist, ausgewählt sind,
R* ein Wasserstoffatom oder ein gegebenenfalls substituierte Alkylgruppe von 1 bis 4 C-Atomen oder ein gegebenenfalls substituierter Arylrest ist,
Y' die Vinylgruppe oder eine Ethylgruppe bedeutet, die in β-Stellung einen durch ein Alkali eliminierbaren Substituenten enthält, oder die β-Hydroxyethyl-Gruppe ist und
A' für einen Rest der allgemeinen Formel (2c) oder (2d)

(2c) 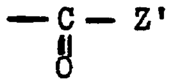 (2d) 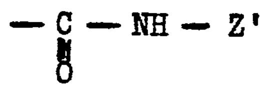

steht, in welchen
Z' ein Phenylrest oder ein Naphthylrest ist, die beide substituiert sein können, bevorzugt ein Rest der Formel (3d), (3e) oder (3f)

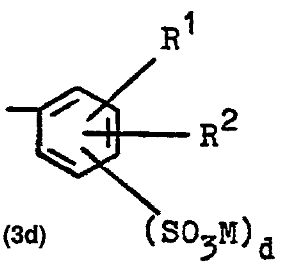 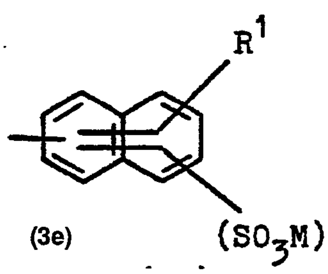 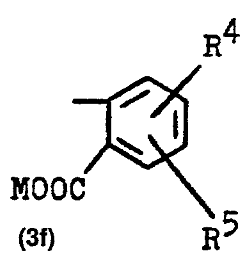

(3d) (3e) (3f)

ist, in welchen
$R^1$, $R^2$ und M die in Anspruch 3 genannten Bedeutungen haben,
d für die Zahl Null, 1 oder 2 steht,
e die Zahl Null, 1, 2 oder 3 bedeutet,
$R^4$ ein Wasserstoffatom, eine Alkylgruppe von 1 bis 4 C-Atomen, eine Alkoxygruppe von 1 bis 4 C-Atomen, ein Chloratom, ein Bromatom oder eine Nitrogruppe und
$R^5$ ein Wasserstoffatom, eine Alkylgruppe von 1 bis 4 C-Atomen, eine Alkoxygruppe von 1 bis 4 C-Atomen oder ein Chloratom ist,
dadurch gekennzeichnet, daß man eine Verbindung der allgemeinen Formel (7)

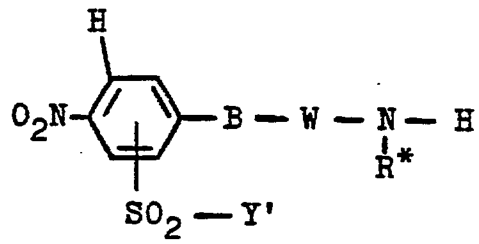

(7)

in welcher Y', B, W und R* die oben genannten Bedeutungen haben, mit einer Verbindung der allgemeinen Formel (8A), (8B) oder (8C)

$Cl - CO - Z'$

(8A)

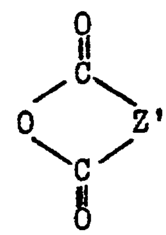

(8B)

$O=C=N - Z'$

(8C)

mit Z' der oben angegebenen Bedeutung, bevorzugt mit einer Verbindung der allgemeinen Formel (8a), (8b), (8c), (9a) oder (9b)

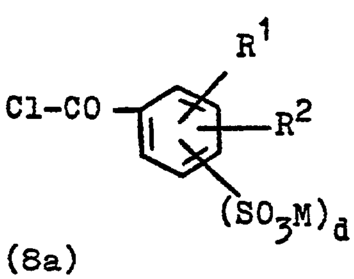

(8a)

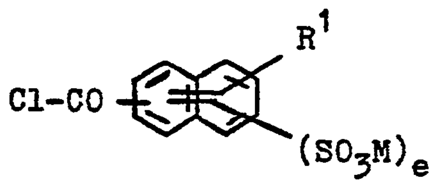

(8b)

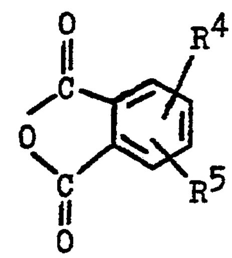

(8c)

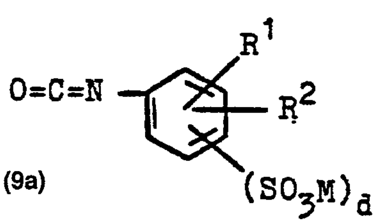

(9a)

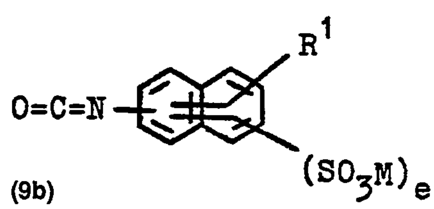

(9b)

in welchen $R^1$, $R^2$, $R^3$, $R^4$, $R^5$, M, d und e die oben genannten Bedeutungen haben, umsetzt und gegebenenfalls in der so erhaltenen Verbindung der allgemeinen Formel (12) mit K der Nitrogruppe die Nitrogruppe zur Aminogruppe reduziert.

## Claims for the Contracting State: ES

1. A process for the preparation of a water-soluble triphendioxazine compound which corresponds to the general formula (1)

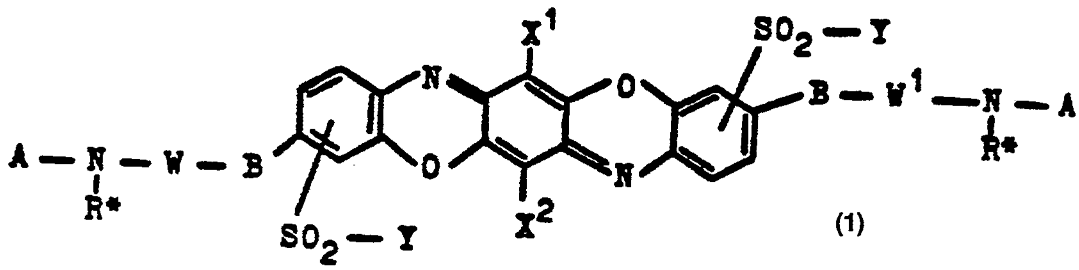

(1)

in which

B is an oxgen or sulfur atom or an amino group of the formula –NH– or –N(R′) in which
R′ is an alkyl group having 1 to 6 carbon atoms;
R* is a hydrogen atom or an optionally substituted alkyl group having 1 to 4 carbon atoms or an optionally substituted aryl radical;
W is a divalent, aliphatic radical or a ($C_5$–$C_{10}$)-cycloaliphatic radical which is optionally substituted by alkyl having 1 to 4 carbon atoms, or an aliphatic-($C_5$–$C_8$)-cycloaliphatic radical which is optionally substituted by alkyl having 1 to 4 carbon atoms, it being possible for the aliphatic radicals to be interrupted by hetero groups selected from the groups –O–, –S–, –$SO_2$–, –CO–,
1,4-piperidino, –NH– and –N(R°)– in which R° has one of the meanings of R′ or is an alkanoyl group having 2 to 5 carbon atoms, and
$W^1$ has one of the meanings indicated for W and is identical with or different from W, or
the grouping –B–$W^1$–N(R*)– and the grouping –N(R*)–W–B–, which are identical with or different from one another, each, as a group, represent the divalent radical of a five-membered or six-membered, saturated heterocyclic structure containing two nitrogen atoms, or
the grouping –B–$W^1$– and the grouping –W–B–, which are identical with or different from one another, each, as a group, represent the divalent radical of a five-membered or six-membered, saturated heterocyclic structure which contains two nitrogen atoms and which is attached by means of one of the two nitrogen atoms, via an alkylene group having 2 to 4 carbon atoms, to the grouping –N(R*)–A or A–N(R*)–, respectively;
A is a radical of the formula (2a) or (2b)

(2a) 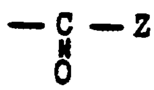 (2b) 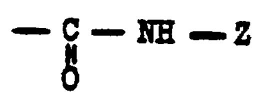

in which

Z is a phenyl radical or a naphthyl radical, both of which are substituted, at least one of these substituents being a group imparting solubility in water;
$X^1$ is a hydrogen atom, a halogen atom, an alkyl group having 1 to 4 carbon atoms, an alkoxy group having 1 to 4 carbon atoms or an optionally substituted aryl radical;
$X^2$ is identical with or different from $X^1$ and has one of the meanings indicated for X1;
Y is the vinyl group or an ethyl group containing, in the β-position, a substituent which can be eliminated by means of an alkali;
the group –$SO_2$–Y is preferably attached in the orthoposition relative to the group –B–W–N(R*)–A or A–N(R*)–W–B–; and
the molecule (1) contains at least two of the sulfo groups which can be present in the molecule (1), which comprises cyclizing a compound of the general fomula (4)

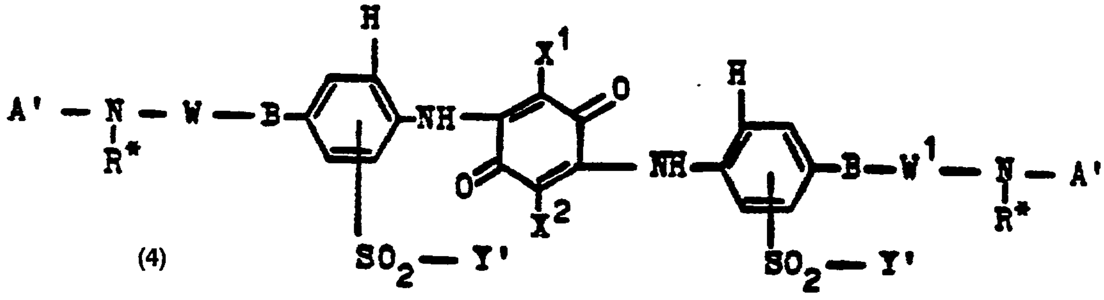

(4)

(in which Y′ is the vinyl group, the β-hydrogethyl group or an ethyl group containing, in the β-position, a substituent which can be eliminated by means of an alkali, such as one of those mentioned for Y, and is preferably the β-hydroxyethyl group, A′ has the meaning of A, but the groups Z do not necessarily have to contain a group inparting solubilityin water, and B, R*, W, $W^1$, $X^1$ and $X^2$ have the abovementioned meanings, and substituted alkyl groups in these radicals can also be hydroxysubstituted alkyl groups, the groups $-SO_2-Y'$ are preferably attached in the ortho-position relative to the group B, and the benzene nuclei must not be substituted in one of the ortho-positions relative to the amino group –NH– indicated) in an acid mediuin and preferablyin the presence of an oxidixing agent to give the triphendioxazine, and, if appropriate, introducing one or more sulfo groups into an aryl radical during the cyclization or after the cyclization.

2. The process as claimed in claim 1, wherein Z is a phenyl radical which is substituted by alkyl having 1 to 4 carbon atoms, alkoxy having 1 to 4 carbon atoms, chlorine, bromine, nitro, carbalkoxy having 2 to 5 carbon atoms, sulfo and/or carboxyl, at least one of these substituents being a sulfo or carboxyl group.

3. The process as claimed in claim 1, wherein Z is a radical of the general formula (3a) or (3b)

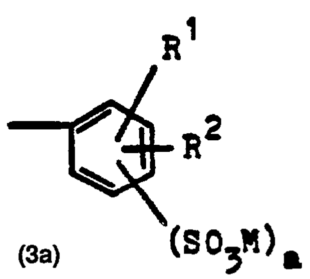

(3a)

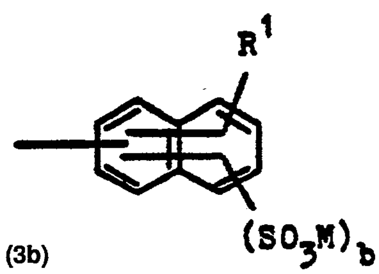

(3b)

in which

M is a hydrogen atom or an alkali metal or the equivalent of an alkaline earth metal,

$R^1$ is a hydrogen atom, an alkyl group having 1 to 4 carbon atoms, an alkoxy group having 1 to 4 carbon atoms, a chlorine atom, a bromine atom, a nitro group, a carbalkoxy group having 2 to 5 carbon atoms or a sulfo group,

$R^2$ is a hydrogen atom, an alkyl group having 1 to 4 carbon atoms, an alkoxy group having 1 to 4 carbon atoms, a chlorine atom, a carboxyl group or a sulfo group,

a represents the number zero or 1, it being necessary for a to be 1 if $R^1$ or $R^2$ does not denote any of the indicated groups imparting solubility in water, and

b denotes the number zero, 1 or 2, it being necessary for b to be the number 1 or 2, if $R^1$ does not represent any of the mentioned groups imparting solubility in water.

4. The process as claimed in claim 3, wherein, in formula (3a), $R^1$ is a hydrogen atom, the methyl, ethyl, methoxy, ethoxy or sulfo group or a chlorine or bromine atom and $R^2$ denotes a hydrogen atom or the methoxy, ethoxy, carboxyl or sulfo group or a chlorine atom, and a represents the number 1.

5. The process as claimed in claim 3, wherein Z is a radical of the formula (3b) in which $R^1$ denotes a hydrogen atom and bis the number 1 or 2.

6. The process as claimed in at least one of claims 1 to 5, wherein W and $W^1$, whioh are identical with one another or different from one another, both denote an alkylene radical which has 2 to 6 carbon atoms and can be interrupted by an oxygen atom or a group -NH-, or both denote a cycloalkylene group having 5 to 6 carbon atoms and having, as appropriate, 1, 2 or 3 methyl groups as substituents, or represent two such cycloalkylene groups attached to an alkylene radical having 1 to 4 carbon atoms, or an alkylene radical which has 2 to 6 carbon atoms and is interrupted by a cycloalkylene group of this type.

7. The process as claimed in at least one of claims 1 to 5, wherein Wand $W^1$ both represent the 1,2-ethylene, 1,3-propylene, 1,4-butylene or isopropylene radical.

8. The process as claimed in at least one of claims 1 to 7, wherein both Y's denote a β-sulfatoethyl group.

9. The process as claimed in at least one of claims 1 to 8, wherein $X^1$ and $X^2$ both denote a chlorine atom or a bromine atom, preferably a chlorine atom.

10. The process as claimed in claim 1, wherein the compound of the general formula (1) is a compound of the general formula (1a)

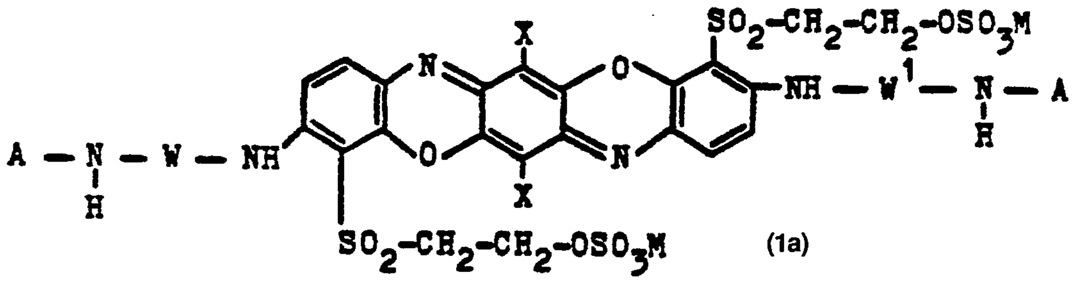

(1a)

in which

W and $W^1$ both denote an alkylene group having 2 to 6 carbon atoms, each

X represents a bromine atom or, particularly preferably, a chlorine atom,

M is a hydrogen atom or an alkali metal and both

A denote a group of the formula (2a) or (2b) mentioned in claim 1 in which the group Z, in each case identical with one another or different from one another, is a group of the general formula (3c)

(3c)

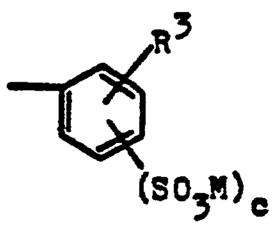

in which

$R^3$ denotes the methyl, ethyl, methoxy or ethoxy group or a sulfo group or a chlorine or bromine atom, and

c represents the number zero, 1 or 2, it being necessary for c to be the number 1 or 2 if $R^3$ does not denote a sulfo group.

11. The process as claimed in claim 1, wherein hydroxyalkyl groups which may be present are converted into sulfatoalkyl or phosphatoalkyl groups at the same time as the cyclization or only after the cyclization, or wherein hydroxyalkyl groups are converted into sulfatoalkyl groups and aryl radicals are sulfonated, at the same time as the cyclization, by means of sulfuric acid or sulfuric acid containing sulfur trioxide, or wherein the sulfonation reaction is carried out after the cyclization at a temperature between 20 and 80°C by means of sulfuric acid or sulfuric acid containing sulfur trioxide, or wherein the cyclization is carried out in sulfuric acid or sulfuric acid containing sulfur trioxide, together with an alkali metal persulfate.

12. The use of a compound of the general formula (1) mentioned and defined in claim 1 or prepared as claimed in claim 1 or 11 for dyeing (including printing) material, in particular fiber material, containing hydroxyl and/or carboxamide groups.

13. A process for dyeing (including printing) material, in particular fiber material, containing hydroxyl and/or carboxamide groups, in which a dyestuff is applied to the material or is introduced into the material and is fixed by means of heat and/or by means of an agent having an alkaline action, which comprises employing a compound of the general formula (1) mentioned and defined in claim 1 or a compound of the formula (1) prepared as claimed in claim 1 or 11 as the dyestuff.

14. A process for the preparation of a compound of the general formula (12)

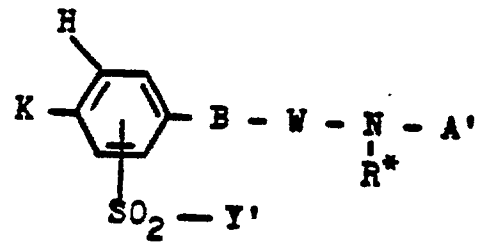

(12)

in which

K is the nitro group or amino group, B is an oxygen or sulfur atom or an amino group of the formula –NH– or –N(R′)– in which

R′ is an alkyl group having 1 to 6 carbon atoms, B thereof is preferably the group –NH–,

W is a divalent, aliphatic radical or a ($C_5$–$C_{10}$)-cycloaliphatic radical which is optionally substituted by alkyl having 1 to 4 carbon atoms, or an aliphatic-($C_5$–$C_8$)-cycloaliphatic radical which is optionally substituted by alkyl having 1 to 4 carbon atoms, it being possible for the aliphatic radicals to be interrupted by hetero groups selected from the groups –O–, –S–, –$SO_2$–, –CO–, 1,4-piperidino, –NH– and –N(R°)– in which R* has one of the meanings of R′, or is an alkanoyl group having 2 to 5 carbon atoms,

R* is a hydrogen atom or an optionally substituted alkyl group having 1 to 4 carbon atoms or an optionally substituted aryl radical,

Y′ denotes the vinyl group or an ethyl group containing, in the β-position, a substituent which can be eliminated by means of an alkali, or is the β-hydroxyethyl group, and

A′ represents a radical of the general formula (2c) or (2d)

(2c) 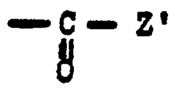 (2d) 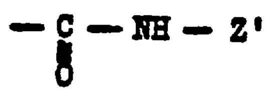

in which Z′ is a phenyl radical or a naphthyl radical, both of which can be substituted, preferably a radical of the formula (3d), (3e) or (3f)

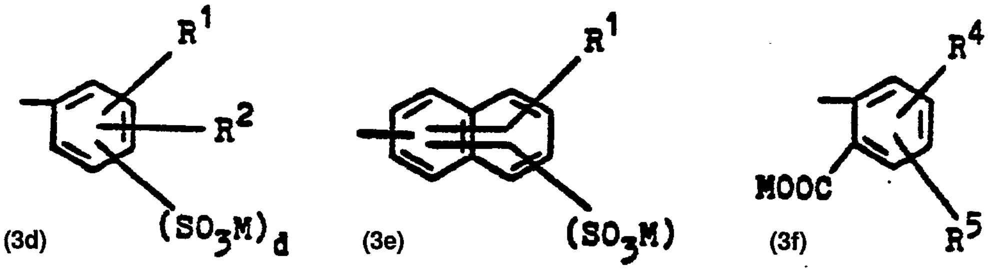

(3d) (3e) (3f)

in which

$R^1$, $R^2$ and M have the meanings mentioned in claim 3, d represents the number zero, 1 or 2, e denotes the number zero, 1, 2 or 3,

$R^4$ is a hydrogen atom, an alkyl group having 1 to 4 carbon atoms, an alkoxy group having 1 to 4 carbon atoms, a chlorine atom, a bromine atom or a nitro group and

$R^5$ is a hydrogen atom, an allgl group having 1 to 4 carbon atoms, an alkoxy group having 1 to 4 carbon atoms or a chlorine atom,

wherein a compound of the general formula (7)

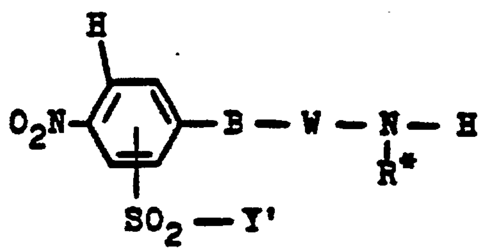

(7)

in which

. Y′, B, W and R* have the meanings mentioned above, is reacted with a compound of the general formula (8A), (8B) or (8C)

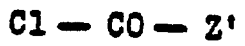

(8A)

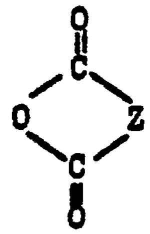

(8B)

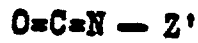

(8C)

where Z′ has the meaning indicated above, preferably with a compound of the general formula (8a), (8b), (8c), (9a) or (9b)

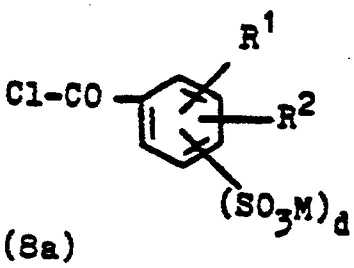

(8a)

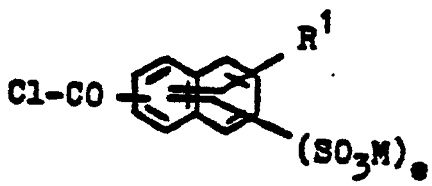

(8b)

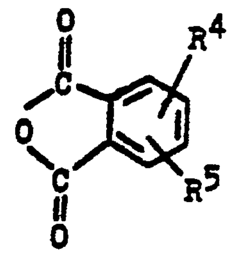

(8c)

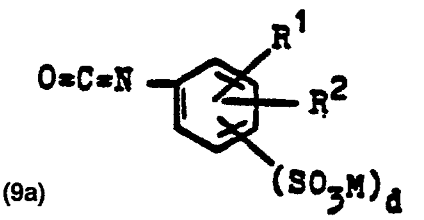

(9a)

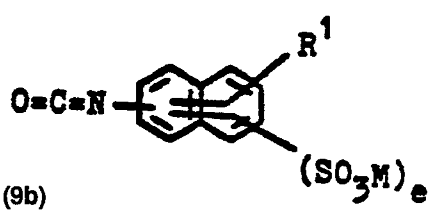

(9b)

in which $R^1$, $R^2$, $R^3$, $R^4$, $R^5$, M, d and e have the abovementioned meanings and, if appropriate, reducing the nitro group in the compound of the general fosnula (12) thus obtained, with K of the nitro group to the amino group.

**Claims for the Contracting States BE, CH, DE, FR, GB, IT, LI**

1. A water-soluble triphendioxazine compound which corresponds to the general formula (1)

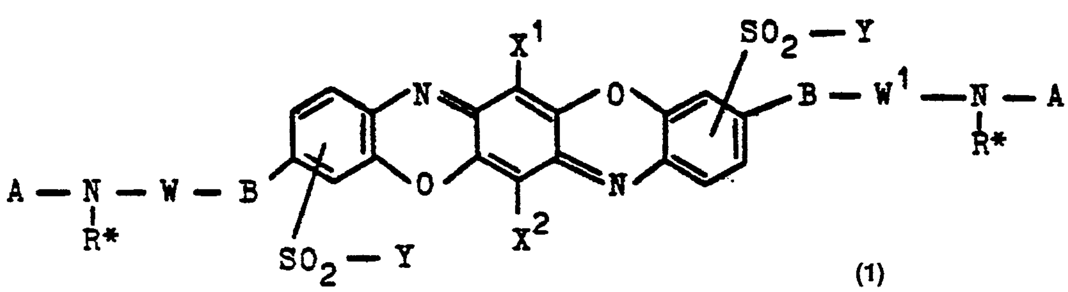

(1)

in which
B is an oxygen or sulfur atom or an amino group of the formula –NH– or –N(R′)–
in which
R′, is an alkyl group having 1 to 6 carbon atoms;
R* is a hydrogen atom or an optionally substituted alkyl group having 1 to 4 carbon atoms or an optionally substituted aryl radical;
W is a divalent, aliphatic radical or a ($C_5$–$C_{10}$))cycloaliphatic radical which is optionally substituted by

alkyl having 1 to 4 carbon atoms, or an aliphatic-($C_5$–$C_8$)-cycloaliphatic radical which is optionally substituted by alkyl having 1 to 4 carbon atoms, it being possible for the aliphatic radicals to be interrupted by hetero groups selected from the groups –O–, –S–, –$SO_2$–, –CO–, 1,4-piperidino, –NH– and –N($R^{\circ}$)– in which $R^{\circ}$ has one of the meanings of R′ or is an alkanoyl group having 2 to 5 carbon atoms, and

$W^1$ has one of the meanings indicated for W and is identical with or different from W, or

the grouping –B–$W^1$–N(R*)– and the grouping –N(R*)–W–B–, which are identical with or different from one another, each, as a group, represent the divalent radical of a five-membered or sixmembered, saturated heterocyclic structure containing two nitrogen atoms, or

the grouping –B–$W^1$– and the grouping –W–B–, which are identical with or different from one another, each, as a group, represent the divalent radical of a five-membered or six-membered, saturated heterocyclic structure which contains two nitrogen atoms and which is attached by means of one of the two nitrogen atoms, via an alkylene group having 2 to 4 carbon atoms, to the grouping –N(R*)–A or A–N(R*)–, respectively;

A is a radical of the formula (2a) or (2b)

(2a) 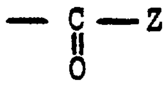 (2b) 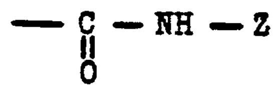

in which

Z is a phenyl radical or a naphthyl radical, both of which are substituted, at least one of these substituents being a group imparting solubility in water;

$X^1$ is a hydrogen atom, a halogen atom, an alkyl group having 1 to 4 carbon atoms, an alkoxy group having 1 to 4 carbon atoms or an optionally substituted aryl radical;

$X^2$ is identical with or different from $X^1$ and has one of the meanings indicated for $X^1$;

Y is the vinyl group or an ethyl group containing, in the β-position, a substituent which can be eliminated by means of an alkali;

the group –$SO_2$–Y is preferably attached in the orthoposition relative to the group –B–W–N(R*)–A or A–N(R*)–W–B–; and the molecule (1) contains at least two of the sulfo groups which can be present in the molecule (1).

2. A compound as claimed in claim 1, wherein Z is a phenyl radical which is substituted by alkyl having 1 to 4 carbon atoms, alkoxy having 1 to 4 carbon atoms, chlorine, bromine, nitro, carbalkoxy having 2 to 5 carbon atoms, sulfo and/or carboxyl, at least one of these substituents being a sulfo or carboxyl group.

3. A compound as claimed in claim 1, wherein Z is a radical of the general formula (3a) or (3b)

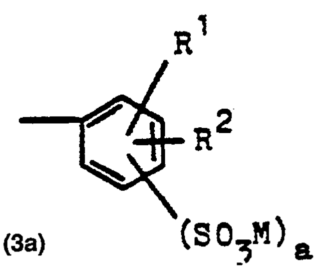
(3a)

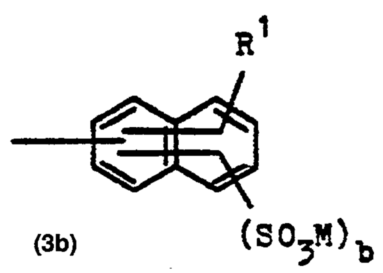
(3b)

in which

M is a hydrogen atom or an alkali metal or the equivalent of an alkaline earth metal,

$R^1$ is a hydrogen atom, an alkyl group having 1 to 4 carbon atoms, an alkoxy group having 1 to 4 carbon atoms, a chlorine atom, a bromine atom, a nitro group, a carbalkoxy group having 2 to 5 carbon atoms or a sulfo group,

$R^2$ is a hydrogen atom, an alkyl group having 1 to 4 carbon atoms, an alkoxy group having 1 to 4 carbon atoms, a chlorine atom, a carboxyl group or a sulfo group,

a represents the number zero or 1, it being necessary for a to be 1 if $R^1$ or $R^2$ does not denote any of the indicated groups imparting solubility in water, and

b denotes the number zero, 1 or 2, it being necessag for b to be the number 1 or 2, if $R^1$ does not represent any of the mentioned groups imparting solubility in water.

4. A compound as claimed in claim 3, wherein, in formula (3a), $R^1$ is a hydrogen atom, the methyl, ethyl, methoxy, ethoxy or sulfo group or a chlorine or bromine atom and $R^2$ denotes a hydrogen atom or the methoxy, ethoxy, carboxyl or sulfo group or a chlorine atom, and a represents the number 1.

5. A compound as claimed in claim 3, wherein Z is a radical of the formula (3b) in which $R^1$ denotes a hydrogen atom and bis the number 1 or 2.

6. A compound as claimed in at least one of claims 1 to 5, wherein Wand $W^1$, which are identical with one another or different from one another, both denote an alkylene radical which has 2 to 6 carbon atoms and can be interrupted by an oxygen atom or a group –NH–, or both denote a cycloalkylene group having 5 or 6 carbon atoms and having, as appropriate, 1, 2 or 3 methyl groups as substituents, or represent two such cycloalkylene groups attached to an alkylene radical having 1 to 4 carbon atoms, or an alkylene radical which has 2 to 6 carbon atoms and is interrupted by a cycloalkylene group of this type.

7. A compound as claimed in at least one of claims 1 to 5, wherein W and $W^1$ both represent the 1,2-ethylene, 1,3-propylene, 1,4-butylene or isopropylene radical.

8. A compound as claimed in at least one of claims 1 to 7, wherein both Y's denote a β-sulfatoethyl group.

9. A compound as claimed in at least one of claims 1 to

8, wherein $X^1$ and $X^2$ both denote a chlorine atom or a bromine atom, preferably a chlorine atom.

10. A compound as claimed in claim 1, of the general formula (Ia),

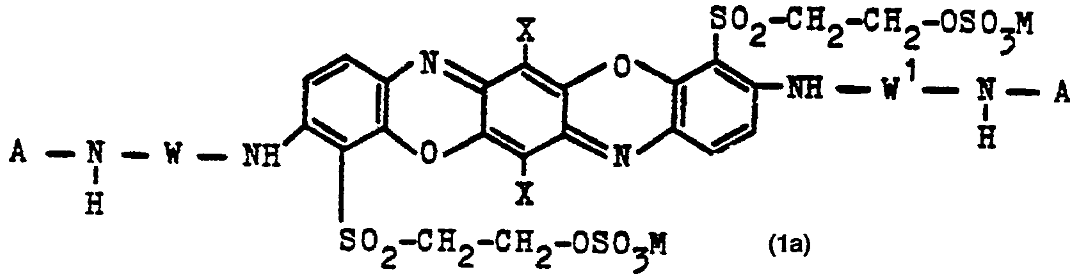
(1a)

in which

W and $W^1$ both denote an alkylene group having 2 to 6 carbon atoms, each

X represents a bromine atom or, particularly preferably, a chlorine atom,

M is a hydrogen atom or an alkali metal and both

A denote a group of the formula (2a) or (2b) mentioned in claim 1 in which the group Z, in each case identical with one another or different from one another, is a group of the general formula (3c)

(3c) 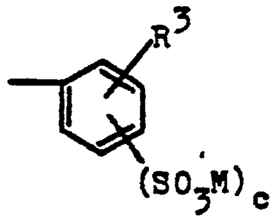

in which

$R^3$ denotes the methyl, ethyl, methoxy or ethoxy group or a sulfo group or a chlorine or bromine atom, and c represents the number zero, 1 or 2, it being necessary for

c to be the number 1 or 2 if $R^3$ does not denote a sulfo group.

11. A process for the preparation of a compound of the general formula (1) mentioned and defined in claim 1, which comprises cyclizing a compound of the general formula (4)

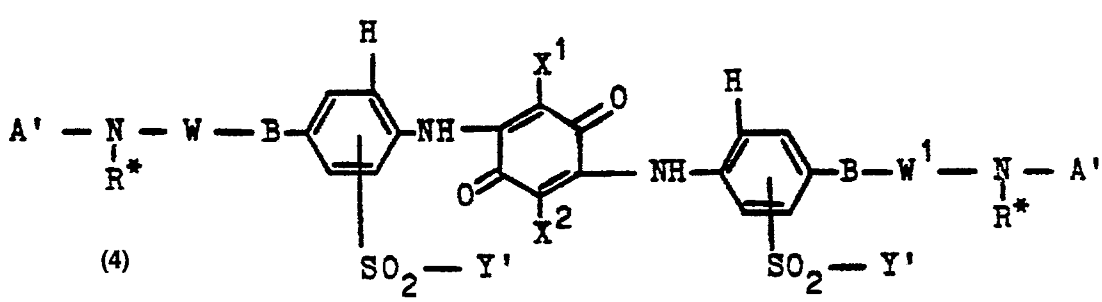
(4)

(in which Y′ is the vinyl group, the β-hydroxyethyl group or an ethyl group containing, in the β-position, a substituent which can be eliminated by means of an alkali, such as one of those mentioned for Y, and is preferably the β-hydroxyethyl group, A′ has the meaning of A, but the groups Z do not necessarily have to contain a group imparting solubility in water, and B, R*, W, $W^1$, $X^1$ and $X^2$ have the meanings mentioned

in claim 1, and substituted alkyl groups in these radicals can also be hydroxysubstituted alkyl groups, the groups $-SO_2-Y'$ are preferably attached in the ortho-position relative to the group B, and the benzene nuclei must not be substituted in one of the ortho-positions relative to the amino group –NH– indicated) in an acid medium and preferablyin the presence of an oxidizing agent to give the triphendioxazine, and, if appropriate, introducing one or more sulfo groups into an aryl radical during the cyclization or after the cyclization.

12. The process as claimedin claim 11, wherein hydroxyalkyl groups which may be present are convertedinto sulfatoalkyl or phosphatoalkyl groups at the same time as the cyclization or only after the cyclization, or wherein hydroxyalkyl groups are converted into sulfatoalkyl groups and aryl radicals are sulfonated, at the same time as the cyclization, by means of sulfuric acid or sulfuric acid containing sulfur trioxide, or wherein the sulfonation reaction is carried out after the cyclization at a temperature between 20 and 80°C by means of sulfuric acid or sulfuric acid containing sulfur trioxide, or wherein the cyclization is carried out in sulfuric acid or sulfuric acid containing sulfur trioxide, together with an alkali metal persulfate.

13. The use of a compound of the general formula (1) mentioned and defined in claim 1 for dyeing (including printing) material, in particular fiber material, containing hydroxyl and/or carboxamide groups.

14. A process for dyeing (including printing) material, in particular fiber material, containing hydroxyl and/or carboxamide groups, in which a dyestuff is applied to the material or is introduced into the material and is fixed by means of heat and/or by means of an agent having an alkaline action, which comprises employing a compound of the general formula (1) mentioned and defined in claim 1 as the dyestuff.

15. A compound of the general formula (12)

(12) 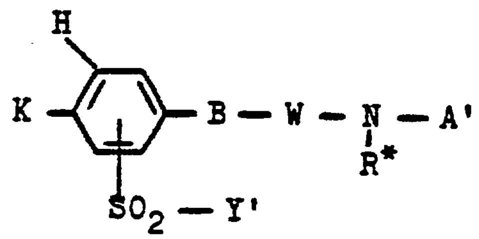

in which

K is the nitro group or ainino group,

B is an oxygen or sulfur atom or an amino group of the formula –NH– or -N(R′)

in which

R′, is an alkyl group having 1 to 6 carbon atoms, B thereof is preferably the group –NH–,

W is a divalent, aliphatic radical or a $(C_3-C_{10})$cycloaliphatic radical which is optionally substituted by alkyl having 1 to 4 carbon atoms, or an aliphatic-$(C_5-C_8)$-cycloaliphatic radical which is optionally substituted by alkyl having 1 to 4 carbon atoms, it being possible for the aliphatic radicals to be interrupted by hetero groups selected from the groups –O–, –S–, $-SO_2-$, –CO–, 1,4-piperidino, –NH– and –N(R°)– in which R° has one of the meanings of R′ or is an alkanoyl group having 2 to 5 carbon atoms,

R* is a hydrogen atom or an optionally substituted alkyl group having 1 to 4 carbon atoms or an optionally substituted aryl radical,

Y′ denotes the vinyl group or an ethyl group containing, in the β-position, a substituent which can be eliminated by means of an alkali, or is the β-hydroxyethyl group, and

A′ represents a radical of the general formula (2c) or (2d)

(2c) 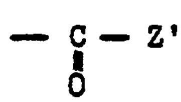 (2d) 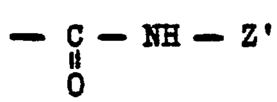

in which Z′ is a phenyl radical or a naphthyl radical, both of which can be substituted, preferably a radical of the formula (3d), (3e) or (3f)

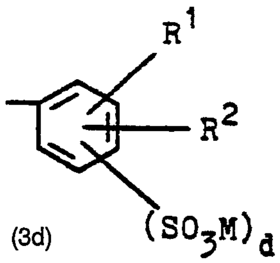

(3d)

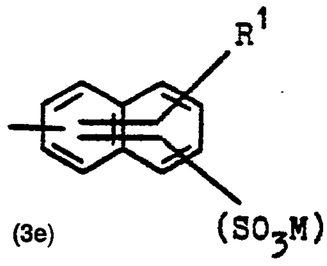

(3e)

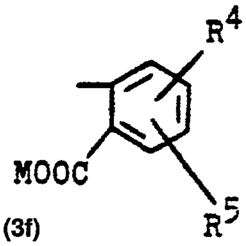

(3f)

in which
$R^1$, $R^2$ and M have the meanings mentioned in claim 3,
d represents the number zero, 1 or 2,
e denotes the number zero, 1, 2 or 3,
$R^4$ is a hydrogen atom, an alkyl group having 1 to 4 carbon atoms, an alkoxy group having 1 to 4 carbon atoms, a chlorine atom, a bromine atom or a nitro group and
$R^5$ is a hydrogen atom, an alkyl group having 1 to 4 carbon atoms, an alkoxy group having 1 to 4 carbon atoms or a chlorine atom.

16. A process for the preparation of a compound of the general formula (12) indicated and defined in claim 15, which comprises reacting a compound of the general formula (7)

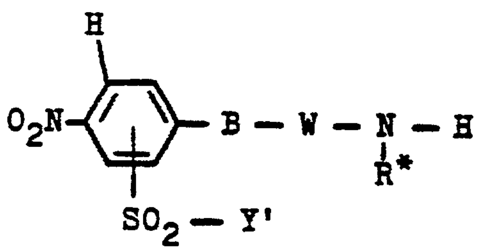

(7)

in which
Y′, B, W and R* have the meanings mentioned in claim 15, with a compound of the general formula (8A), (8B) or (8C)

$Cl - CO - Z'$

(8A)

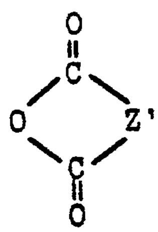

(8B)

$O{=}C{=}N - Z'$

(8C)

in which Z′, has the meaning indicated in claim 15, preferably with a compound of the general formula (8a), (8b), (8c), (9a) or (9b)

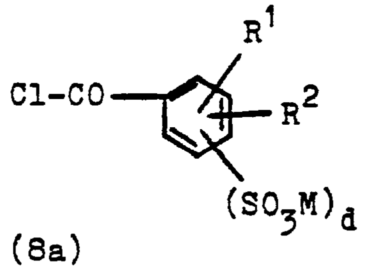

(8a)

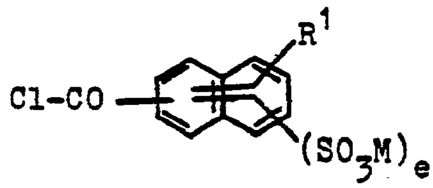

(8b)

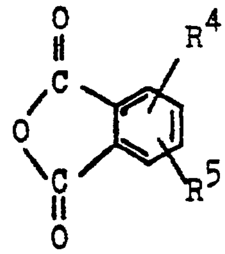

(8c)

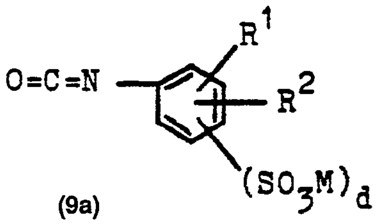

(9a)

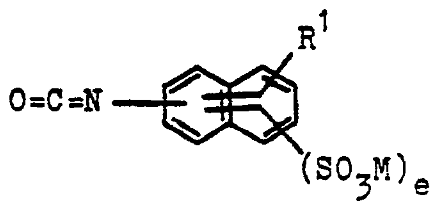

(9b)

in which $R^1$, $R^2$, $R^3$, $R^4$, $R^5$, M, d and e have the meanings mentioned in claim 15 and, if appropriate, reducing the nitro group in the compound of the general formula (12) thus obtained, with K of the nitro group to the amino group.

17. The use of a compound of the general formula (12) of claim 15 for the synthesis of dyestuffs, in particular triphendioxazine compounds of the general formula (1) as claimed in claim 1.

**Revendications pour l'Etat Contractent: ES**

1. Procédé de préparation de composés triphénodioxaziniques hydrosolubles de formule générale 1:

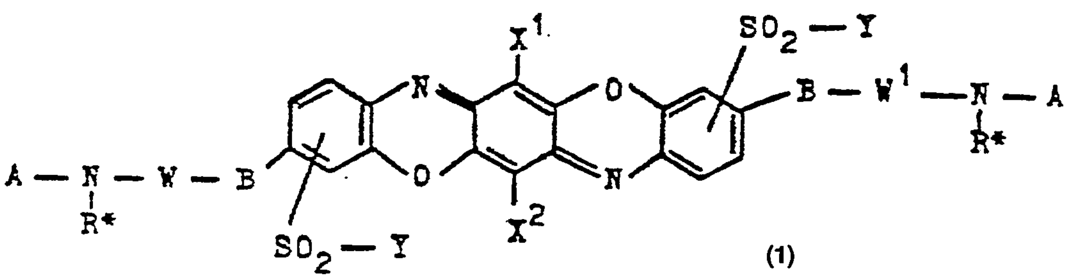

(1)

dans laquelle le symbole B désigne un atome d'oxygène ou de soufre ou un groupe amino -NH- ou -N'(R'), R' étant un alkyle en $C_1$ à $C_6$;

R* désigne un atome d'hydrogène ou bien un alkyle en $C_1$ à $C_4$ ou un aryle éventuellement substitués;

W un radical bivalent, aliphatique ou bien cycloaliphatique en $C_5$–$C_{10}$ avec éventuellement un substituant alkylique en $C_1$ à $C_4$ ou encore aliphatico-($C_5$–$C_8$)-cycloaliphatique avec aussi éventuellement un substituant alkylique en $C_1$ à $C_4$, les radicaux aliphatiques pouvant être interrompus par des hétéro-atomes ou des hétéro-groupes; tels que -O-, -S-, $-SO_2-$, -CO-, 1, 4-pypéridino, -NH- et - N($R^0$)-,

$R^0$ ayant l'une des significations de R' ou étant un alcanoyle en $C_2$–$C_5$, et

$W^1$ a l'une des significations données pour W et est identique à W ou différent de W, ou bien

le groupement -B-$W^1$-N(R*)- et le groupement -N(R*)-W-B-, qui peuvent être identiques ou différents l'un de l'autre, représentent chacun le radical bivalent d'un hétérocycle saturé pentagonal ou hexagonal à deux atomes d'azote, ou les groupements -B-$W^1$- et -W-B-, qui peuvent être aussi identiques ou différents l'un de l'autre, représentent chacun le radical bivalent d'un hétérocycle saturé pentagonal ou hexagonal à deux atomes d'azote, lié par l'un des deux atomes d'azote au groupement -N(R*)-A ou A-N(R*)- par l'intermédiaire d'un alkylène en C2 à C4;

A est un radical de formule 2a ou 2b

(2a) 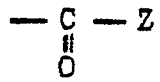 (2b) 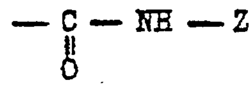

Z désignant un phényle ou un naphtyle substitués, dont l'un au moins des substituants est un groupe hydrosolubilisant;

$X^1$ est un atome d'hydrogène ou d'halogène ou un alkyle ou un alcoxy en $C_1$–$C_4$ ou un aryle éventuellement substitué;

$X^2$ est identique à $X^1$ ou lui est différent et a l'une des significations indiquées pour $X^1$;

Y est le groupe vinyle ou un groupe éthyle avec à une position β un substituant éliminable par un alcali;

les deux groupes $-SO_2-Y$ occupent chacun de préférence la position ortho par rapport aux groupes respectifs $-B-W-N(R^*)-A$ et $A-N(R^*)-W^1-B-$ ; et la molécule (1), qui a au moins deux groupes sulfo, peut en avoir plus de deux.

Procédé caractérisé en ce que l'on cyclise en une triphénodioxazine, en milieu acide et de préférence en présence d'un agent d'oxydation, un composé de formule générale 4:

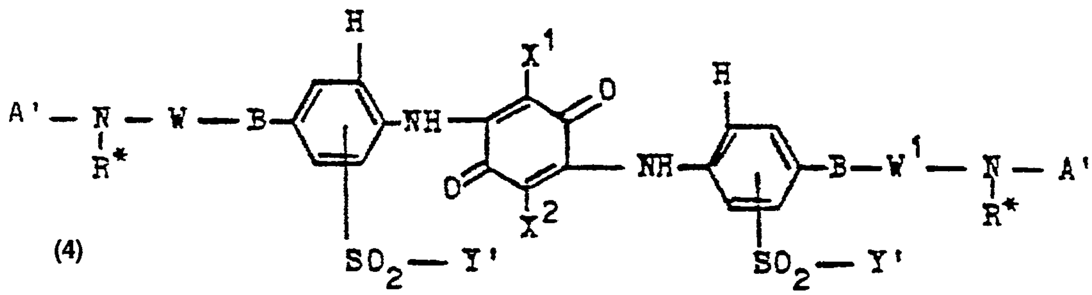

(4)

(dans laquelle Y′ est le groupe vinyle ou β-hydroxyéthyle ou un groupe éthyle ayant à la position β un substituant éliminable par un alcali, tel qu'indiqué pour Y, mais de préférence le groupe β-hydroxyéthyle, A′ a la même signification que A, les groupes Z toutefois ne devant pas être obligatoirement substitués par un groupe hydrosolubilisant, et B, R*, W, $W^1$, $X^1$ et $X^2$ ont les significations précédemment données, des alkyles substitués de ces radicaux pouvant être également des hydroxyalkyles, les groupes $-SO^2$-Y′ sont de préférence à une position ortho par rapport au groupe B et les cycles benzéniques ne doivent pas avoir de substituant à l'une des positions ortho par rapport au groupe amino -NH-), et le cas échéant, au cours de la cyclisation et après celle-ci, on fixe un ou plusieurs groupes sulfo sur un radical aryle.

2. Procédé selon la revendication 1, caractérisé en ce que Z est un phényle avec des substituants pris parmi des alkyles et des alcoxy en $C_1$–$C_4$, le chlore et le brome et des groupes nitro, alcoxycarbonyles en $C_2$–$C_5$, sulfo et/ou carboxy, l'un au moins de ces substituants étant un groupe hydrosolubilisant sulfo ou carboxy.

3. Procédé selon la revendication 1, caractérisé en ce que Z est un radical de formule 3a ou 3b:

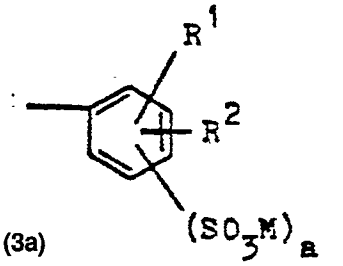

(3a)

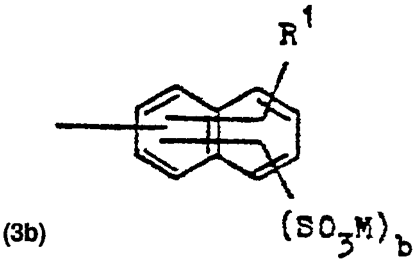

(3b)

dans lesquelles

M représente un atome d'hydrogène ou un métal alcalin ou l'équivalent d'un métal alcalino-terreux;

$R^1$ représente un atome d'hydrogène, un alkyle ou un alcoxy en $C_1$ à $C_4$, un atome de chlore ou de brome ou un groupe nitro, alcoxycarbonyle en $C_2$ à $C_5$, ou un groupe sulfo, et $R^2$ un atome d'hydrogène, un alkyle en $C_1$ à $C_4$, un alcoxy en $C_1$ à $C_4$, un atome de chlore ou un groupe carboxy ou sulfo,

a est le nombre 0 ou 1 mais obligatoirement le nombre 1 si $R^1$ ou $R^2$ n'est pas l'un des groupes hydrosolubilisant indiqués à la revendication 2, et

b le nombre 0, 1 ou 2 mais obligatoirement le nombre 1 ou 2 si $R^1$ n'est pas l'un des groupes hydrosolubilisants indiqués.

4. Procédé selon la revendication 3, caractérisé en ce que dans la formule 3a $R^1$ est un atome d'hydrogène, le groupe méthyle, éthyle, méthoxy, éthoxy ou sulfo ou bien un atome de chlore ou de brome et $R^2$ un atome d'hydrogène, le groupe méthoxy, éthoxy, carboxy ou sulfo ou un atome de chlore, et a le nombre 1.

5. Procédé selon la revendication 3, caractérisé en ce que Z est un radical de formule 3b, $R^1$ étant un atome d'hydrogène et b le nombre 1 ou 2.

6. Procédé selon une ou plusieurs des revendications 1 à 5, caractérisé en ce que W et $W^1$, identiques entre eux ou différents l'un de l'autre, sont des alkylènes en $C_2$–$C_6$ pouvant être interrompus par un atome d'oxygène ou un groupe -NH-, ou des cycloalkylènes en $C_5$ ou $C_6$ avec éventuellement comme substituants 1, 2 ou 3 groupes méthyle ou encore deux tels cycloalkylènes reliés par un alkylène en $C_1$–$C_4$, ou bien des alkylènes en $C_2$–$C_6$ interrompus par un tel cycloalkylène.

7. Procédé selon une ou plusieurs des revendications 1 à 5, caractérisé en ce que W et $W^1$ sont chacun le groupe 1, 2-éthylène, 1, 3-propylène, 1, 4-butylène ou isopropylène.

8. Procédé selon une ou plusieurs des revendications 1 à 7, caractérisé en ce que les deux Y sont des groupes β-sulfatoéthyles.

9. Procédé selon une ou plusieurs des revendications 1 à 8, caractérisé en ce que $X^1$ et $X^2$ sont des des atomes de chlore ou de brome, de préférence de chlore.

10. Procédé selon la revendication 1, caractérisé en ce que le composé de formule 1 est un composé de formule 1a:

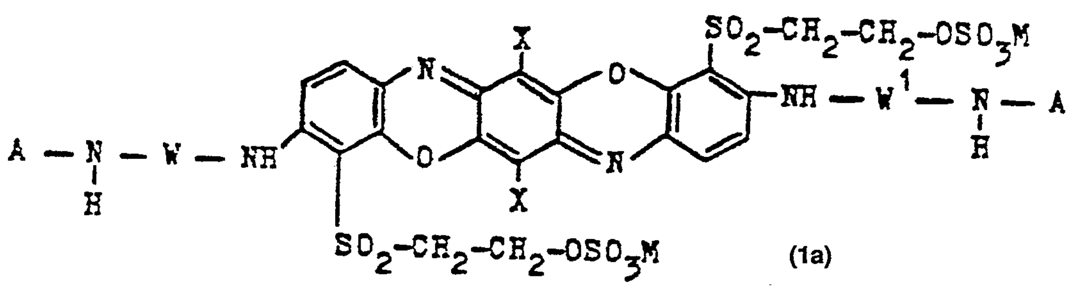

(1a)

dans laquelle

W et $W^1$ sont des alkylènes en $C_2$–$C_6$, les deux symboles X désignent des atomes de brome ou de préférence de chlore,

M est un atome d'hydrogène ou un métal alcalin, et les deux symboles A désignent chacun un groupe de formule 2a ou 2b de la revendication 1 dont les radicaux Z, identiques ou différents l'un de l'autre, sont chacun un radical de formule 3c:

(3c) 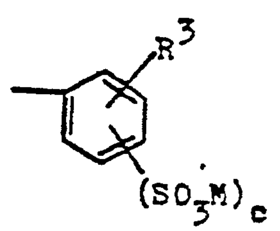

$R^3$ étant le groupe méthyle, éthyle, méthoxy ou éthoxy ou bien un groupe sulfo ou un atome de chlore ou de brome, et

c le nombre 0, 1 ou 2 mais obligatoirement le nombre 1 ou 2 si $R^3$ n'est pas un groupe sulfo.

11. Procédé selon la revendication 1, caractérisé en ce que, en même temps que la cyclisation ou seulement après celle-ci, on transforme des groupes hydroxyalkyliques éventuellement présents en groupes sulfatoalkyliques ou phosphato-alkyliques, ou bien 5 en même temps que la cyclisation on transforme des groupes hydroxyalkyliques en groupes sulfatoalkyliques et on sulfone des radicaux aryliques avec de l'acide sulfurique ou de l'acide sulfurique contenant du trioxyde de soufre, ou bien on effectue la réaction de sulfonation après la cyclisation à une température de 20 à 80°C avec de l'acide sulfurique ou de l'acide sulfurique contenant du trioxyde de soufre, ou encore on effectue la cyclisation dans de l'acide sulfurique contenant éventuellement du trioxyde de soufre avec un persulfate de métal alcalin.

12. L'emploi des composés de formule générale 1 définis à la revendication 1 ou qui ont été obtenus suivant la revendication 1 ou 11 pour la teinture, comprenant aussi l'impression, de matières ayant des groupes hydroxyliques et/ou carboxamides en particulier de matières fibreuses.

13. Procédé de teinture, comprenant également l'impression, de matières à groupes hydroxyliques

-et/ou carboxamides, notamment de watières fibreuses, par application d'un colorant sur la matière ou incorporation du colorant dans celle-ci et fixation du colorant sous l'action de la chaleur et/ou d'un agent alcalin, procédé caractérisé en ce que l'on utilise comme colorant un composé de formule générale 1 définie à la revenùication 1 ou qui a été obtenu suivant la revendication 1 ou 11.

14. Procédé de préparation des composés de formule 12:

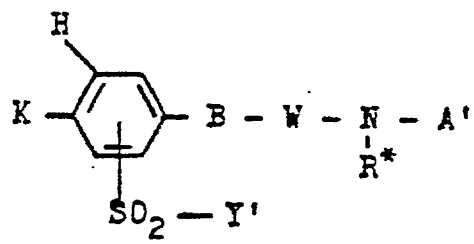

(12)

dans laquelle

K est le groupe nitro ou amino,

B un atome d'oxygène ou de soufre ou un groupe amino -NH- ou -N(R')-,

R' étant un alkyle en $C_1$–$C_6$,

B étant de préférence le groupe -NH-,

W un radical bivalent, aliphatique, cycloaliphatique en $C_5$–$C_{10}$ éventuellement substitué par un alkyle en $C_1$–$C_4$ ou aliphatico-($C_5$–$C_8$)-cycloaliphatique éventuellement substitué aussi par un alkyle en $C_1$–$C_4$, les radicaux aliphatiques pouvant être interrompus par des hétéroatomes ou des hétéro-groupes pris parmi les suivants : -O-, -S-, $-SO_2-$, -CO-, 1, 4-pipéridino, -NH- et $-N(R^0)-$, $R^0$ ayant l'une des significations de R', ou étant un alcanoyle en $C_2$–$C_5$,

R* est un atome d'hydrogène ou bien un alkyle en $C_1$–$C_4$ ou un aryle éventuellement substitués,

Y' le groupe vinyle ou un groupe éthyle avec à la position β un substituant éliminable par un alcali, ou le groupe β-hydroxyéthyle, et A, un radical de formule 2c ou 2d:

(2c) 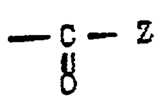 (2d) 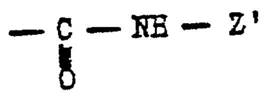

dans lesquelles

Z', est un phényle ou un naphtyle pouvant être substitués, de préférence un radical de formule 3d, 3e ou 3f :

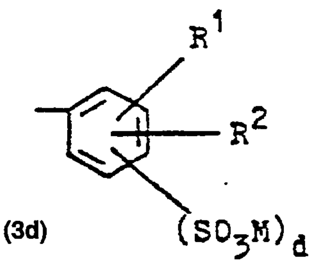 (3d) 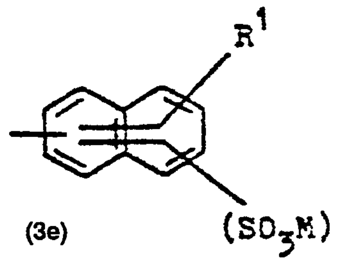 (3e) 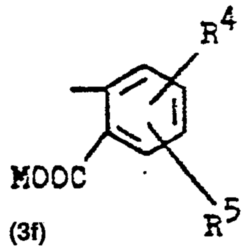 (3f)

formules dans lesquelles $R^1$, $R^2$ et Mont les significations données à la revendication 3,

d est le nombre 0, 1 ou 2,

e le nombre 0, 1, 2 ou 3,

$R^4$ un atome d'hydrogène, un alkyle ou un alcoxy en $C_1$–$C_4$ ou bien un atome de chlore ou de brome ou un groupe nitro, et $R^5$ un atome d'hydrogène, un alkyle ou un alcoxy en $C_1$–$C_4$ ou un atome de chlore, procédé caractérisé en ce que l'on fait réagir un composé de formule générale 7:

(7) 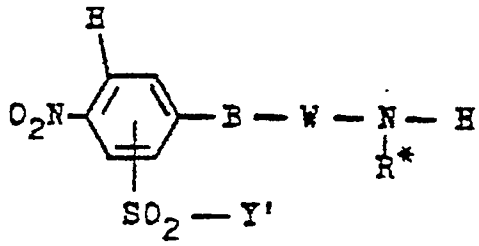

avec un composé de formule 8A, 8B ou 8C :

$Cl-CO-Z'$

(8A)

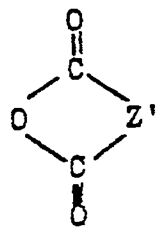

(8B)

$O=C=N-Z'$

(8C)

de préférence avec un composé de formule 8a, 8b, 8c, 9a ou 9b:

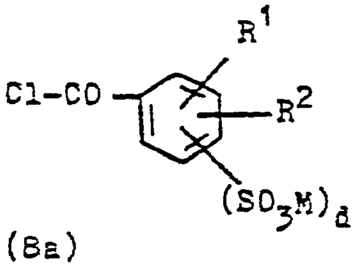

(8a)

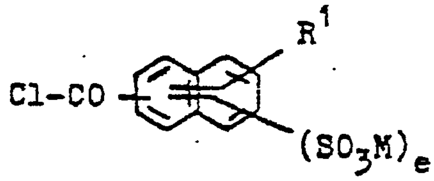

(8b)

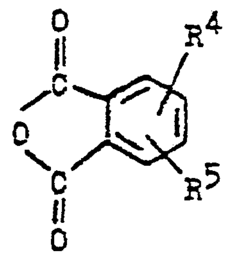

(8c)

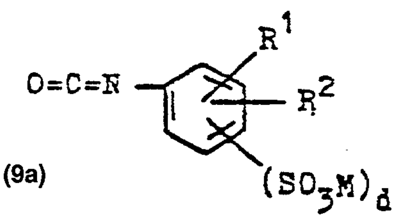

(9a)

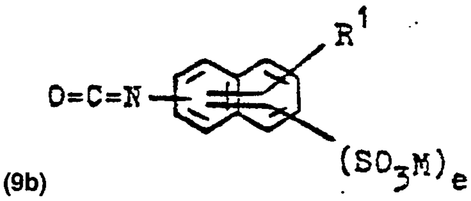

(9b)

et le cas échéant, si dans le composé de formule 12 ainsi obtenu K est le groupe nitro, on le réduit en groupe amino.

**Revendications pour les Etats Contractants: BE, CH, DE, FR, GB, IT, LI**

1. Les composés triphénodioxaziniques hydrosolubles de formule générale 1 ci-dessous:

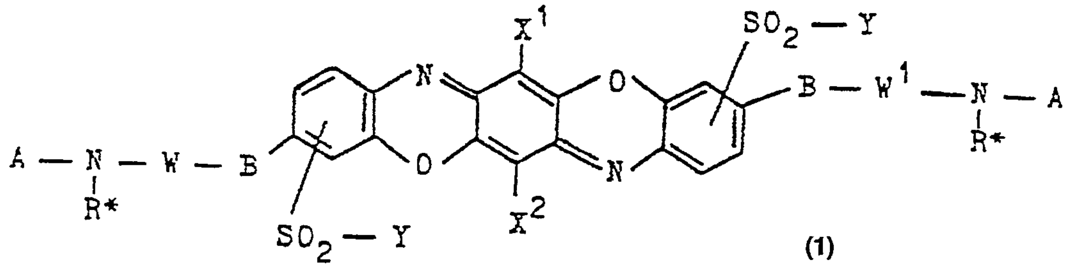

(1)

dans laquelle

le symbole B désigne un atome d'oxygène ou de soufre ou un groupe amino -NH- ou -N(R')-,

R' étant un alkyle en $C_1$ à $C_6$;

R* désigne un atome d'hydrogène ou bien un alkyle en $C_1$ à $C_4$ ou un aryle éventuellement substitués;

W un radical bivalent, aliphatique ou bien cycloaliphatique en $C_5$–$C_{10}$ avec éventuellement un substituant alkylique en $C_1$ à $C_4$ ou encore aliphatico-($C_5$–$C_8$)-cycloaliphatique avec aussi éventuellement un substituant alkylique en $C_1$ à $C_4$, les radicaux aliphatiques pouvant être interrompus par des hétéro-atomes ou des hétéro-groupes , tels que -O-, -S-, $-SO_2-$, -CO-, 1, 4-pypéridino, -NH- et $-N(R^0)-$,

$R^0$ ayant l'une des significations de R, ou étant un alcanoyle en $C^2$–$C^5$, et

$W^1$ a l'une des significations données pour W et est identique à W ou différent de W, ou bien

le groupement $-B-W^1-N(R^*)-$ et le groupement -N(R*)-W-B-, qui peuvent être identiques ou différents l'un de l'autre, représentent chacun le radical bivalent d'un hétérocycle saturé pentagonal ou hexagonal à deux atomes d'azote, ou

les groupements $-B-W^1-$ et -W-B-, qui peuvent être aussi identiques ou différents l'un de l'autre, représentent chacun le radical bivalent d'un hétérocycle saturé pentagonal ou hexagonal à deux atomes d'azote, lié par l'un des deux atomes d'azote au groupement -N(R*)-A ou A-N(R*)- par l'intermédiaire d'un alkylène en $C_2$ à $C_4$;

A est un radical de formule 2a ou 2b

(2a) 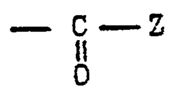 (2b) 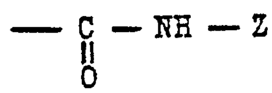

Z désignant un phényle ou un naphtyle substitués, dont l'un au moins des substituants est un groupe hydrosolubilisant;

$X^1$ est un atome d'hydrogène ou d'halogène ou un alkyle ou un alcoxy en $C_1$–$C_4$ ou un aryle éventuellement substitué;

$X^2$ est identique à $X^1$ ou lui est différent et a l'une des significations indiquées pour $X^1$;

Y est le groupe vinyle ou un groupe éthyle avec à une position β un substituant éliminable par un alcali;

les deux groupes $-SO_2-Y$ occupent chacun de préférence la position ortho par rapport aux groupes respectifs -B-W-N(R*)-A et $A-N(R^*)-W^1-B-$;

et la molécule (1), qui a au moins deux groupes sulfo, peut en avoir plus de deux.

2. Composé selon la revendication 1 caractérisé en ce que Z est un radical phényle avec des substituants pris parmi des alkyles et alcoxy en $C_1$–$C_4$, le chlore et le brome et des groupes nitro, alcoxycarbonyles en $C_2$–$C_5$, sulfo et/ou carboxy, l'un au moins de ces substituants étant un groupe hydrosolubilisant sulfo ou carboxy.

3. Composé selon la revendication 1 caractérisé en ce que Z est un radical de formule 3a ou 3b :

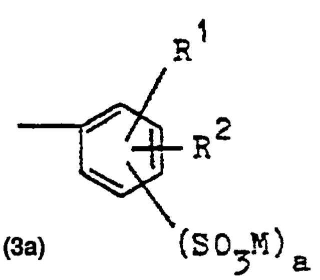

(3a)

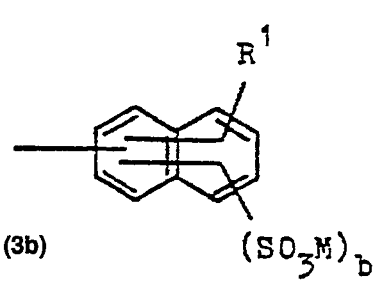

(3b)

dans lesquelles

M représente un atome d'hydrogène ou un métal alcalin ou l'équivalent d'un métal alcalino-terreux;

$R^1$ représente un atome d'hydrogène, un alkyle ou un alcoxy en $C_1$ à $C_4$, un atome de chlore ou de brome ou un groupe nitro, alcoxycarbonyle en $C_2$ à $C_5$, ou un groupe sulfo, et

$R^2$ un atome d'hydrogène, un alkyle en $C_1$ à $C_4$, un alcoxy en $C_1$ à $C_4$, un atome de chlore ou un groupe carboxy ou sulfo,

a est le nombre 0 ou 1 mais obligatoirement le nombre 1 si $R^1$ ou $R^2$ n'est pas l'un des groupes hydrosolubilisant indiqués à la revendication 2, et

b le nombre 0, 1 ou 2 mais obligatoirement le nombre 1 ou 2 si $R^1$ n'est pas l'un des groupes hydrosolubilisants indiqués.

4. Composé selon la revendication 3, caractérisé en ce que dans la formule 3a $R^1$ est un atome d'hydrogène ou un groupe méthyle, éthyle, méthoxy, éthoxy ou sulfo ou encore un atome de chlore ou de brome et R2 un atome d'hydrogène, un groupe méthoxy, éthoxy, carboxy ou sulfo ou un atome de chlore, et a est le nombre 1.

5. Composé selon la revendication 3 caractérisé en ce que Z est un radical de formule 3b dont $R^1$ est un atome d'hydrogène et b le nombre 1 ou 2.

6. Composé selon une ou plusieurs des revendications 1 à 5, caractérisé en ce que W et $W^1$, qui peuvent être identiques ou différents l'un de l'autre, sont des alkylènes en $C_2$–$C_6$ pouvant être interrompus par un atome d'oxygène ou un groupe -NH-, ou bien des cycloalkylènes en $C_5$ ou $C_6$ avec éventuellement comme substituants 1, 2 ou 3 groupes méthyle ou encore deux tels cycloalkylènes reliés par un alkylène en $C_1$–$C_4$ ou des alkylènes en $C_2$–$C_6$ interrompus par un tel cycloalkylène.

7. Composé selon une ou plusieurs des revendications 1 à 5, caractérisé en ce que W et $W^1$ sont chacun le groupe 1, 2-éthylène, 1, 3-propylène, 1, 4-butylène ou isopropylène.

8. Composé selon une ou plusieurs des revendications 1 à 7, caractérisé en ce que les deux Y sont des groupes β-sulfatoéthyles.

9. Composé selon une ou plusieurs des revendications 1 à 8, caractérisé en ce que $X^1$ et $X^2$ sont chacun un atome de chlore ou de brome, de préférence de chlore.

10. Composés selon la revendication 1, de formule générale 1a:

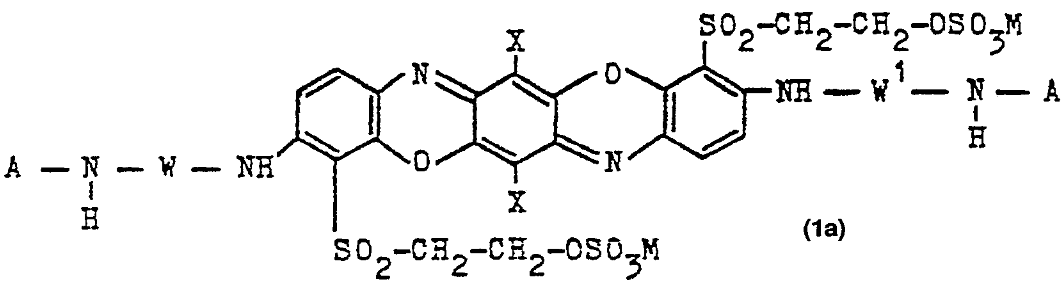

(1a)

dans laquelle

W et $W^1$ sont des alkylènes en $C_2$–$C_6$,

les deux symboles X désignent des atomes de brome ou de préférence de chlore,

M est un atome d'hydrogène ou un métal alcalin, et

les deux symboles A désignent chacun un groupe de formule 2a ou 2b de la revendication 1 dont les radicaux Z, identiqnes ou différents l'un de l'autre, sont chacun un radical de formule 3c:

(3c) 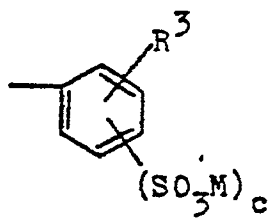

$R^3$ étant le groupe méthyle, éthyle, méthoxy ou éthoxy ou bien un groupe sulfo ou un atome de chlore ou de brome, et

c le nombre 0, 1 ou 2 mais obligatoirement le nombre 1 ou 2 si $R^3$ n'est pas un groupe sulfo.

11. Procédé de préparation des composés de formule 1 définis à la revendication 1, procédé caractérisé en ce que l'on cyclise en une triphénodioxazine, en milieu acide et de préférence en présence d'un agent d'oxydation, un composé de formule générale 4 :

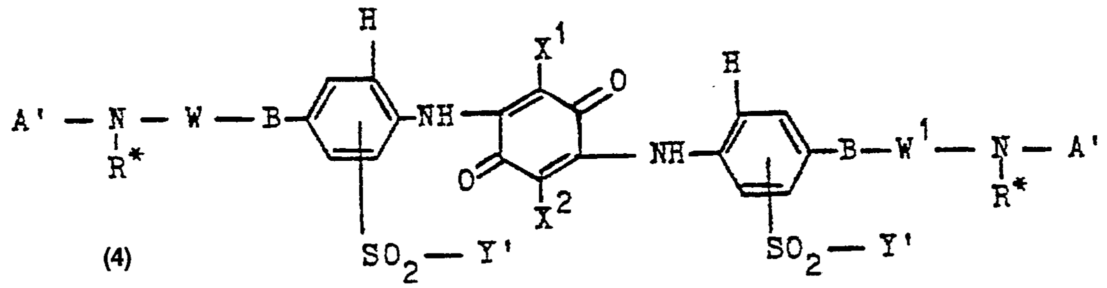

(4)

(dans laquelle Y′ est le groupe vinyle ou β-hydroxyéthyle ou un groupe éthyle ayant à la position b un substituant éliminable par un alcali, tel qu'indiqué pour Y, mais de préférence le groupe β-hydroxyéthyle, A′ a la même signification que A, les groupes Z toutefois ne devant pas être obligatoirement substitués par un groupe hydrosolubilisant, et B, R*, W, $W^1$, $X^1$ et $X^2$ ont les significations données à la revendication 1, des alkyles substitués de ces radicaux pouvant être également des hydroxy-alkyles, les groupes -$SO_2$-Y, sont de préférence à une position ortho par rapport au groupe B et les cycles benzéniques ne doivent pas avoir de substituant à l'une des positions ortho par rapport au groupe amino -NH-), et le cas échéant, au cours de la cyclisation ou après celle-ci, on fixe un ou plusieurs groupes sulfo sur un radical aryle.

12. Procédé selon la revendication 11, caractérisé en ce que, en même temps que la cyclisation ou seulement après celle-ci, on transforme des groupes hydroxyalkyliques éventuellement présents en groupes sulfatoalkyliques ou phosphato-alkyliques, ou bien en même temps que la cyclisation on transforme des groupes 5 hydroxyalkyliques en groupes sulfatoalkyliques et on sulfone des radicaux aryliques avec de l'acide sulfurique ou de l'acide sulfurique contenant du trioxyde de soufre, ou bien on effectue la réaction de sulfonation après la cyclisation à une température de 20 à 80°C avec de l'acide sulfurique ou de l'acide sulfurique contenant du trioxyde de soufre, ou encore on effectue la cyclisation dans de l'acide sulfurique contenant éventuellement du trioxyde de soufre avec un persulfate de métal alcalin.

13. L'emploi des composés de formule 1 définis à la revendication 1 pour la teinture, y compris l'impression, de matières ayant des groupes hydroxyliques et/ou carboxamides, en particulier de matières fibreuses.

14. Procédé de teinture, y compris l'impression, de matières à groupes hydroxyliques et/ou carboxamides, en particulier de matières fibreuses, par application sur la matière ou incorporation dans celle-ci d'un colorant et fixation de celui-ci sous l'action de la chaleur et/ou d'un agent alcalin, procédé caractérisé en ce que l'on utilise comme colorant un composé de formule 1 selon la revendication 1.

15. Les composés de formule générale 12:

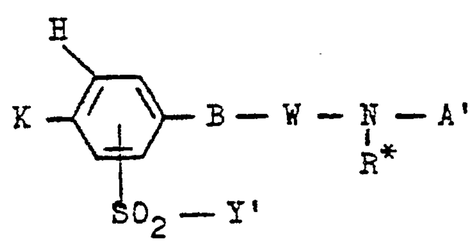

(12)

dans laquelle

K est le groupe nitro ou amino,

B un atome d'oxygène ou de soufre ou un groupe amino -NH ou -N(R′)-,

R′ étant un alkyle en $C_1$–$C_6$,

B étant de préférence le groupe -NH-,

W un radical bivalent, aliphatique, cycloaliphatique en $C_5$–$C_{10}$ éventuellement substitué par un alkyle en $C_1$–$C_4$ ou aliphatico-($C_5$–$C_8$)-cycloaliphatique éventuellement substitué aussi par un alkyle en $C_1$–$C_4$, les radicaux aliphatiques pouvant être interrompus par des hétéroatomes ou des hétéro-groupes pris parmi les suivants:

-0-, -S-, $SO_2$-, -CO-, 1, 4-pipéridino, -NH- et - N($R^0$)-, $R^0$ ayant l'une des significations de R′ ou étant un alcanoyle en $C_2$–$C_5$,

R* est un atome d'hydrogène ou bien un alkyle en $C_1$–$C_4$ ou un aryle éventuellement substitués,

Y′ le groupe vinyle ou un groupe éthyle avec à la position β un substituant éliminable par un alcali, ou le groupe β-hydroxyéthyle, et

A′ un radical de formule 2c ou 2d :

(2c) 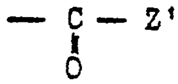 (2d) 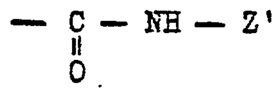

dans lesquelles Z, est un phényle ou un naphtyle pouvant être substitués, de préférence un radical de formule 3d, 3e ou 3f :

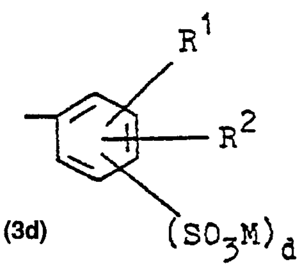
(3d)

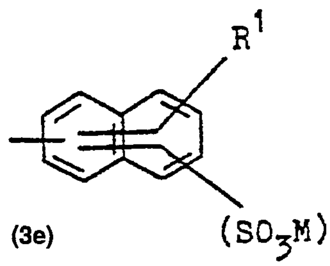
(3e)

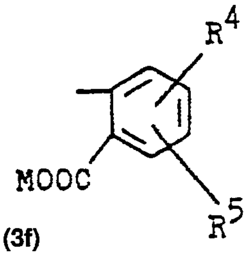
(3f)

formules dans lesquelles

$R^1$, $R^2$ et Mont les significations données à la revendication 3,

d est le nombre 0, 1 ou 2,

e le nombre 0, 1, 2 ou 3,

$R^4$ un atome d'hydrogène, un alkyle ou un alcoxy en $C_1$–$C_4$ ou bien un atome de chlore ou de brome ou un groupe nitro, et

$R^5$ un atome d'hydrogène, un alkyle ou un alcoxy en $C_1$–$C_4$ ou un atome de chlore.

16. Procédé de préparation des composés de formule 12 tels que définis à la revendication 15, procédé caractérisé en ce que l'on fait réagir un composé de formule générale 7:

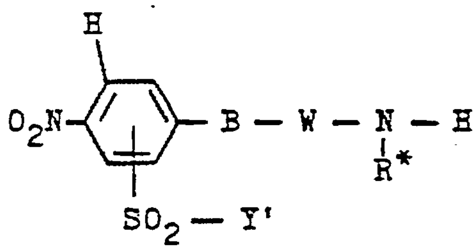
(7)

avec un composé de formule 8A, 8B ou 8C :

$Cl — CO — Z'$

(8A)

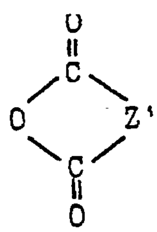
(8B)

$O=C=N — Z'$

(8C)

de préférence avec un composé de formule 8a, 8b, 8c, 9a ou 9b:

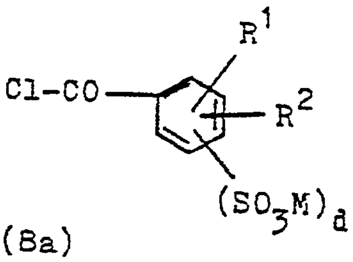

(8a)

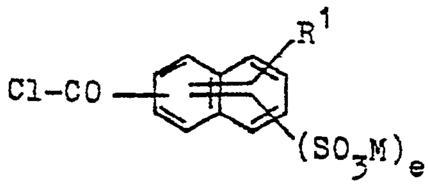

(8b)

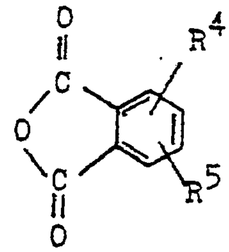

(8c)

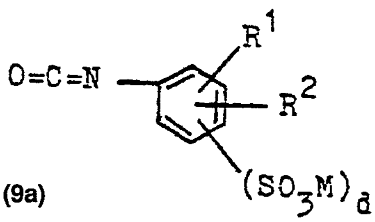

(9a)

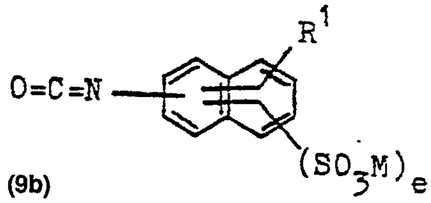

(9b)

et le cas échéant, si dans le composé de formule 12 ainsi obtenu K est le groupe nitro, on réduit ce groupe en un groupe amino.

17. L'emploi des composés de formule 12 de la revendication 15 pour la synthèse de colorants, en particulier de composés triphénodioxaziniques de formule générale 1 selon la revendication 1.